Europäisches Patentamt

**European Patent Office**

Office européen des brevets

(11) Numéro de publication: **0 268 501 B1**

(12) FASCICULE DE BREVET EUROPEEN

(45) Date de publication de fascicule du brevet: **19.01.94**   (51) Int. Cl.⁵: **C12N 15/00**, C07K 13/00, C12N 1/20, C12N 7/00, C12N 5/00, C12P 21/02, A61K 37/02, C07K 15/00, C12N 9/10, A61K 39/00

(21) Numéro de dépôt: **87401915.1**

(22) Date de dépôt: **20.08.87**

(54) **Protéines à activité glutathion-S-transférase, séquences d'ADN, anticorps, poxvirus et médicaments pour la prévention de la schistosomose.**

(30) Priorité: **22.08.86 FR 8611986**
      **22.04.87 FR 8705691**
      **22.04.87 FR 8705692**

(43) Date de publication de la demande:
      **25.05.88 Bulletin  88/21**

(45) Mention de la délivrance du brevet:
      **19.01.94 Bulletin  94/03**

(84) Etats contractants désignés:
      **AT BE CH DE ES FR GB GR IT LI LU NL SE**

(56) Documents cités:
      **EP-A- 0 110 385**

      **MOLECULAR AND BIOCHEMICAL PARASITO-LOGY, vol. 18, no. 1, janvier 1986, pages 73-88, Elsevier Science Publishers B.V. (BIO-MEDICAL DIVISION); J.S. CORDINGLEY et al.: "Identification by message selection of cDNA clones encoding antigens of Schisto-soma mansoni"**

(73) Titulaire: **TRANSGENE S.A.**
      **11, rue de Molsheim**
      **F-67000 Strasbourg(FR)**

(72) Inventeur: **Sondermeyer, Paul**
      **32, rue Raphael**
      **F-67200 Strasbourg(FR)**
      Inventeur: **Balloul, Jean-Marc**
      **51, rue Bonte Pollet**
      **F-59000 Lille(FR)**
      Inventeur: **Pierce, Raymond**
      **23, rue de Flandre**
      **F-59113 Seclin(FR)**
      Inventeur: **Grzych, Jean-Marie**
      **1, rue Meunier**
      **Apt. 202**
      **F-59700 Marcq en Baroeul(FR)**
      Inventeur: **Kieny, Marie-Paule**
      **1, rue de Gascogne**
      **F-67100 Strasbourg(FR)**

Idem

PROCEEDINGS OF THE NATIONAL ACADEMY OF SCIENCES OF THE USA, vol. 83, novembre 1986, pages 8703-8707, D.B. SMITH et al.: "Mr 26,000 antigen of Schistosoma japonicum recognized by resistant WEHI 129/J mice is a parasite glutathione S-transferase"

MOLECULAR AND BIOCHEMICAL PARASITOLOGY, vol. 17, no. 1, janvier 1985, pages 105-114, Elsevier Science Publishers B.V. (Biomedical Division); J.-M. BALLOUL et al.: "In vitro synthesis of a 28 kilodalton antigen present on the surface of the Schistosomulum of Schistosoma mansoni"

NATURE, vol. 326, 12 mars 1987, pages 149-153; J.M. BALLOUL et al.: "Molecular cloning of a protective antigen of schistosomes

PROCEEDINGS OF THE NATIONAL ACADEMY OF SCIENCES OF THE USA, vol. 83, août 1986, pages 5534-5538; A.H. DAVIS et al.: "Isolation of cDNA clones for differentially expressed genes of the human parasite Schistosoma mansoni"

Inventeur: **Loison, Gérard**
**19, rue du Fg National**
**F-67000 Strasbourg(FR)**
Inventeur: **Capron, André**
**58 rue du Capitaine Jasmin**
**F-59113 Phalempin(FR)**
Inventeur: **Lecocq, Jean-Pierre**
**6, rue du Champ du Feu**
**F-67116 Reichstett(FR)**

(74) Mandataire: **Ahner, Francis et al**
**CABINET REGIMBEAU**
**26, avenue Kléber**
**F-75116 Paris (FR)**

**Description**

La présente invention concerne la mise au point d'un vaccin contre la schistosomose.

La schistosomose (ou bilharziose) est une maladie parasitaire des régions tropicales et sub-tropicales du monde entier (à l'exception de l'Amérique du Nord) ; elle s'étend même en Afrique du Nord ; il en existe un important foyer en Egypte et quelques foyers ont été signalés en Espagne et au Portugal.

Cette maladie affecte de 200 à 400 millions d'êtres humains. Le parasite responsable, le schistosome, est un petit ver plat dont le cycle de vie complexe passe par un hôte intermédiaire qui est un mollusque d'eau douce. Pour cette raison, on voit la maladie s'étendre avec la création des barrages et des réseaux d'irrigation destinés à la mise en valeur des contrées tropicales.

On connait 5 types de schistosomes pathogènes pour l'homme : le plus répandu, S. mansoni, est commun à l'Afrique et l'Amérique ; 2 espèces, S. haematobium et intercalatum, se retrouvent seulement en Afrique et 2 autres, S. japonicum et mekongi, en Asie.

La connaissance du cycle de vie du parasite est nécessaire à la mise au point d'une stratégie de prévention de la maladie.

En effet, le parasite présente une adaptation quasi-parfaite aux défenses naturelles de l'hôte : quand il atteint le stade adulte, il échappe totalement aux mécanismes immunitaires et peut vivre de 5 à 20 ans; dans les vaisseaux sanguins proches de la paroi intestinale ou de la vessie, selon les espèces (S. mansoni ou S. haematobium respectivement). Le parasite ne se multiplie pas chez l'homme mais la femelle pond jusqu'à 3 000 oeufs par jour. Ces oeufs et les déchets du métabolisme du ver se retrouvent dans la circulation de l'hôte et provoquent des troubles divers (troubles intestinaux, urinaires, affaiblissement, anémie, formation de granulomes avec blocage des vaisseaux sanguins capillaires entraînant des fibroses tissulaires et en particulier des inflammations hépatiques graves).

Les oeufs, pourvus d'un éperon, peuvent traverser la paroi intestinale ou vésicale où ils provoquent des microlésions, puis ils sont éliminés. S'ils arrivent rapidement au contact de l'eau, ils y libèrent des larves ciliées (miradicium) qui nagent jusqu'à ce qu'elles pénètrent dans un mollusque. Dans cet hôte intermédiaire, la larve poursuit des cycles de multiplication asexuée qui donneront naissance à des cercaires qui seront libérées dans l'eau. (Un mollusque infecté par un seul miracidium peut produire plus de 50 000 cercaires en une vie de 8 à 10 mois).

La cercaire est une larve nageante qui ne vit que 1 à 2 jours. Quand elle rencontre un mammifère, l'homme en particulier, elle pénètre au travers de sa peau. Dans la peau, elle se transforme en schistosomule, forme immature du parasite, qui va être entraîné dans la circulation sanguine, successivement vers le coeur, les poumons et enfin le foie. C'est dans la circulation hépatique que le parasite devient adulte. Le mâle et la femelle s'apparient ; la femelle s'installe dans un canal formé par le reploiement du corps du mâle et le couple se fixe définitivement dans un vaisseau sanguin abdominal où la femelle commence à pondre.

La maturation complète du schistosomule en ver adulte prend une quinzaine de jours. Au stade adulte, le parasite a adsorbé à sa surface des molécules de son hôte et, grâce à ce camouflage, il échappe à ses mécanismes de reconnaissance et de défense immunitaires. De plus, le parasite sécrète des substances qui ont un pouvoir immunosuppresseur. Ainsi protégé, chaque couple de schistosomes peut vivre dans son hôte pendant de nombreuses années en pondant des milliers d'oeufs chaque jour.

Il existe un médicament efficace contre le parasite adulte, le Praziquantel, mais il coûte trop cher pour qu'on puisse envisager son usage généralisé dans les pays en voie de développement. De plus, il n'empêche pas la réinfection, or, en zone endémique, les populations sont en contact régulier avec des eaux contaminées par les larves.

Le seul espoir sérieux de prévention de la maladie ne peut venir que de la mise au point d'un vaccin efficace contre la forme immature du parasite, le schistosomule. Ce vaccin devrait être administré aux jeunes enfants pour éviter l'infection primaire et aux individus déjà contaminés, après un traitement au Praziquantel pour prévenir la réinfection.

Ce vaccin devrait contenir un (ou des) antigène(s) majeur(s) du schistosomule et induire une réaction immunitaire efficace contre la larve avant qu'elle n'échappe aux mécanismes de défense de l'hôte.

Des travaux récents (voir revue par Capron et Dessaint - 1985) ont permis d'identifier un nombre limité d'antigènes protéiques présents à la surface du parasite et de sa larve et qui pourraient jouer un rôle important dans l'induction d'une réponse immunitaire.

Des antigènes ont été mis en évidence par Balloul et al. (1985) : après immunisation de rats par des extraits de S. mansoni purifiés sur gel, les anticorps induits reconnaissent un groupe de trois antigènes de 28 Kd, qui est présent sur les formes adultes et larvaire du parasite. Ces mêmes anticorps reconnaissent un produit de traduction in vitro des $RNA_m$ extraits du parasite adulte et une des protéines externes du

schistosomule, identifiée par marquage à l'$^{125}$I.

Ces anticorps anti-p28, bien qu'ils ne puissent pas par eux-mêmes neutraliser le schistosomule, activent la réponse cellulaire cytotoxique qui peut tuer les schistosomules.

Un échantillon de la protéine p28 purifiée a été inoculé à des rats et des souris ; les animaux immunisés ont développé un haut degré de protection contre une infection expérimentale par les schistosomules (Balloul et al. 1986).

Cet ensemble d'observations met en évidence l'importance de l'antigène p28 dans l'induction d'une réponse immunitaire protectrice.

La présente invention concerne l'identification et la détermination de la séquence du cADN qui code pour l'antigène p28 ou au moins pour un polypeptide qui comprend les épitopes de p28 qui sont reconnus par des anticorps dressés contre la protéine p28 native.

La présente invention a tout d'abord pour objet une protéine selon l'une des revendications 1 à 3.

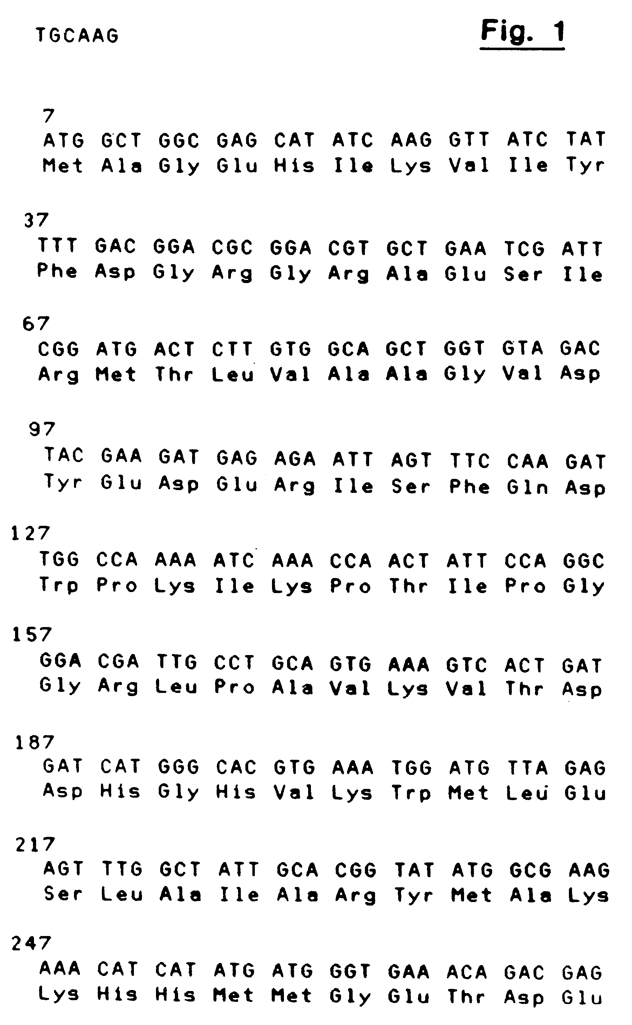

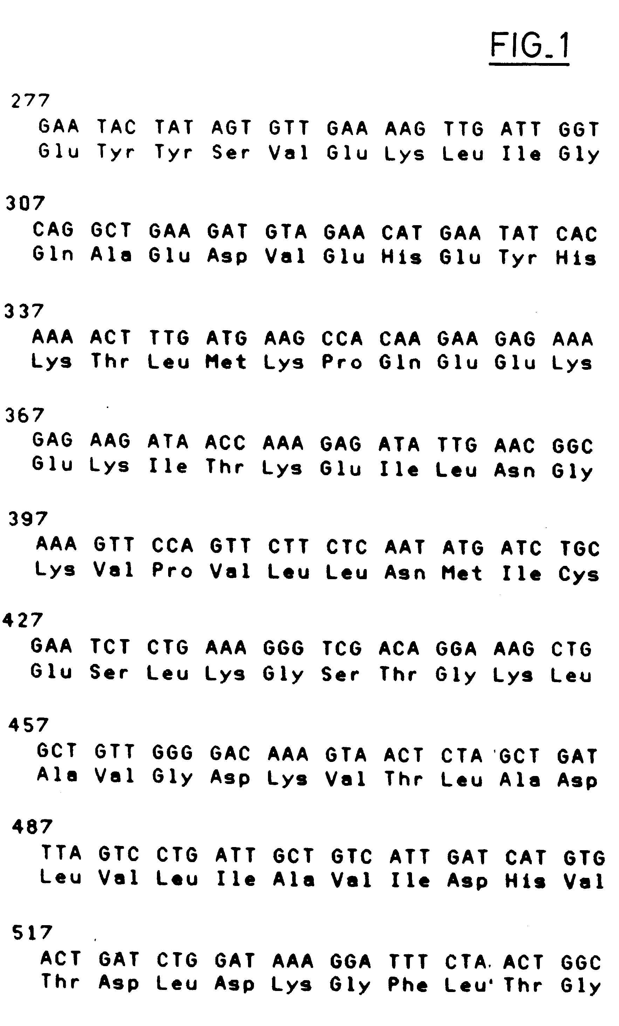

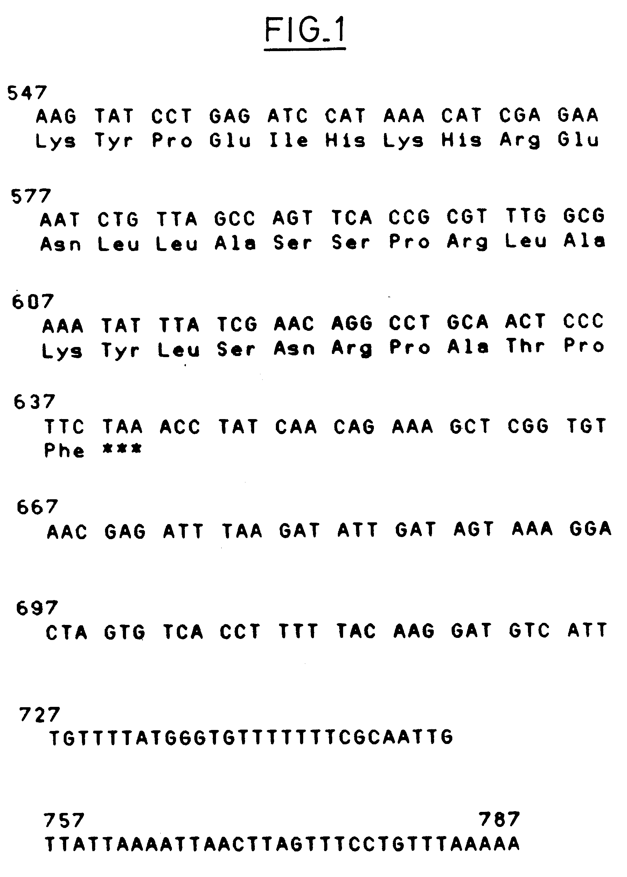

La présente invention concerne également la séquence d'ADN codant pour la protéine p28 mature tel que décrit à la figure 1.

La présente invention concerne également la protéine dont la synthèse est dirigée par ce cADN, synthèse qui peut être effectuée dans différentes cellules hôtes, bactéries, levures ou cellules supérieures, en fonction du vecteur dans lequel le cADN est inséré et des signaux de contrôle de l'expression sous lesquels il est placé.

La protéine selon la présente invention peut présenter une structure primaire identique à celle de la protéine p28 native, présente dans le schistosomule, mais elle peut aussi être un dérivé de celle-ci ou une protéine p28 incomplète mais comprenant les épitopes importants pour la reconnaissance par les anticorps et donc pour l'induction de l'immunité. Cette protéine ou la protéine incomplète peut encore être fusionnée à une autre protéine (ou fragment de protéine), suite à une manipulation génétique des segments d'ADN correspondants, destinée à favoriser une meilleure expression de la protéine dans la cellule hôte ou éventuellement à provoquer son excrétion hors de la cellule.

Les technologies permettant le clonage et l'expression d'un gène étranger dans différentes cellules hôtes sont connues de l'homme de métier. Elles seront illustrées dans les exemples ci-après, étant entendu que d'autres vecteurs et d'autres cellules hôtes peuvent être utilisés.

L'expression du gène correspondant à p28 dans une bactérie, en l'occurence E.coli, a été obtenue avec un vecteur comprenant le promoteur de lambdaP$_L$ et le CIIrbs comme site de fixation des ribosomes. La protéine hybride obtenue comporte une partie de la protéine CII et la protéine p28 ou une partie de celle-ci.

Dans le cas où l'hôte est une levure, par exemple S. cerevisiae, il est possible d'utiliser un vecteur plasmidique de levure comportant une origine de réplication fonctionnelle dans la levure, par exemple l'origine de réplication du plasmide 2μ et, un gène de sélection tel que URA3. Dans le cas présent, le promoteur utilisé est celui du gène PGK avec la partie 5' du gène PGK qui conduit soit à une protéine fusionnée, soit à une protéine mature.

Enfin, lorsque l'hôte est une cellule de mammifère, on utilisera de préférence comme vecteur d'expression un poxvirus, par exemple le virus de la vaccine dans lequel le gène codant pour la protéine sera placé sous le contrôle d'une séquence d'expression de ce gène par le poxvirus. Ce virus recombinant comportera le gène codant pour une protéine selon l'invention inséré de préférence dans le gène TK de la vaccine et sous le contrôle d'un promoteur fort tel que le promoteur de la protéine 7,5 K de la vaccine. Le gène codant pour la protéine selon l'invention pourra également être fusionné à une région codant pour une séquence signal fonctionnelle, par exemple la séquence signal de l'interleukine 2 ou de la glycoprotéine rabique, pour assurer l'excrétion de la protéine hors de la cellule, dans ce cas la protéine correspondante sera une protéine hybride comportant une partie de protéine non originaire des schistosomules.

Le gène codant pour la protéine selon l'invention pourra également être fusionné à une région codant pour la zone transmembranaire de la glycoprotéine du virus de la rage pour assurer l'ancrage de la protéine dans la membrane cytoplasmique des cellules infectées.

L'invention concerne également les cellules hôtes transformées par les vecteurs d'expression des protéines selon la présente invention ainsi qu'un procédé de préparation de telles protéines, caractérisé en ce qu'on cultive ces cellules hôtes et que l'on récupère les protéines selon l'invention.

Bien entendu, les méthodes de culture, de récupération et de séparation des protéines dépendront de l'hôte mais sont connues de l'homme de métier.

Enfin, la présente invention concerne des compositions pharmaceutiques utiles comme vaccins contre la schistosomose, caractérisés en ce qu'ils comportent au moins une protéine ou un poxvirus selon l'invention dans un support pharmaceutiquement acceptable.

La présente invention concerne en particulier des vaccins vivants, caractérisés en ce qu'ils comportent des virus de la vaccine recombinants vivants qui expriment la protéine selon l'invention, présentés dans un support pharmaceutiquement acceptable.

De préférence, ces vaccins seront sous forme administrable par exemple par injection. Le support pharmaceutiquement acceptable pourra être un support aqueux pour injection.

Le processus de vaccination devra être adapté au type de vaccin utilisé (protéine ou virus vivant).

Enfin, la présente invention concerne des antisérums dressés contre une protéine selon la présente invention.

La présente invention concerne en outre la mise en évidence d'une activité enzymatique de glutathion-S-tranférase de la protéine p28 recombinante (ou p28I).

Les glutathion-S-transférases sont des enzymes très largement distribuées, présentes aussi bien chez les végétaux que chez les animaux et qui jouent un rôle dans la détoxification de diverses molécules (composés électrophiles hydrophobes, superoxydes endogènes, agents alkylants, herbicides...).

Le travail de Smith et al. (1986) montre que le parasite Schistosoma japonicum synthétise une glutathion-S-transférase qui pourrait jouer un rôle dans la protection du parasite contre les radicaux libres produits par les cellules effectrices de l'immunité de l'hôte infecté.

La présente invention concerne plus particulièrement la mise en évidence d'une activité de glutathion-S-transférase de la protéine p28I dérivée du Schistosoma mansoni et synthétisée dans E. coli ou S. cerevisiae, et dont l'intérêt comme antigène vaccinal a été montré ci-dessus.

Plus particulièrement, la présente invention concerne l'application d'une protéine telle que décrite ci-dessus à titre d'agent présentant une activité glutathion-S-transférase telle que la protéine p28.

La présente invention concerne également l'application des cellules, transformées ou infectées par un bloc d'expression de la protéine précédemment décrite, à titre d'agent présentant une activité glutathion-S-transférase.

Ces protéines et ces cellules peuvent être utilisées comme agent de conversion dans un procédé de conversion microbiologique mettant en oeuvre une activité glutathion-S-transférase.

La présente invention concerne également la préparation desdites protéines par culture des cellules décrites précédemment.

Enfin, l'invention concerne la composition pharmaceutique pour le traitement ou la prévention de la schistosomose, caractérisée en ce qu'elle comporte à titre d'agent actif au moins une protéine telle que décrite précédemment.

Bien entendu, si le cADN est incorporé dans un poxvirus tel que la vaccine, ce poxvirus pourra dans certains cas être utilisé directement comme agent vaccinant.

Il est également possible de dresser des anticorps contre ces protéines. Ces anticorps et ces protéines pourront être utilisés comme agent de diagnostic de la schistosomose.

Dans la présente invention les séquences de nucléotides des gènes ou d'acides aminés des protéines qui sont représentés dans les figures ne seront pas reprises mais sont partie intégrante de l'invention et de la présente description.

Les exemples ci-après sont destinés à mettre en évidence d'autres caractéristiques et avantages de la présente invention.

La description sera faite en utilisant les figures ci-annexées.

Les figures ci-annexées sont les suivantes :

- La figure 1 représente la séquence complète du cADN déduite des séquences de λTG06, λTG08, λTG09, λTG10 et λTG11.

  La phase ouverte de lecture qui commence au 7ème nucléotide code pour un polypeptide de 211 acides aminés, d'un poids moléculaire de 28 kd.

- La figure 2 représente la construction de pTG44 par intégration d'un insert EcoRI de λTG06 dans pTG1924. pTG1924 est un dérivé de pTG908 avec un site EcoRI mis en phase, situé à 46 pb en aval du codon initiateur de CII. pTG44 code pour une protéine fusionnée cII/p28 d'un poids moléculaire de 25 kd.

- La figure 3 représente la séquence de la protéine de fusion cII/p28 codée par le bloc d'expression inséré dans pTG44.

- La figure 4 représente des extraits totaux et fractions insolubles des extraits de E. coli TGE901 pTG44 après électrophorèse en gel d'acrylamide-SDS et coloration au bleu de Coomassie.

  La flèche indique la position de la protéine de 25 kd.

  A. extrait total de TGE901 pTG44 à 30°C

  B. fraction non soluble du même extrait

  C. extrait total de TGE901 pTG44 après induction pendant 7 heures à 37°C

  D. fraction non soluble du même extrait

  E. extrait total de TGE901 pTG44 après induction pendant 7 heures à 42°C

F. fraction non soluble du même extrait

G. fraction F. solubilisée en SDS 0,2 %

H. extrait total de culture contrôle de TGE901 pTG[1924] après 7 heures à 42°C

I. fraction non soluble du même extrait.

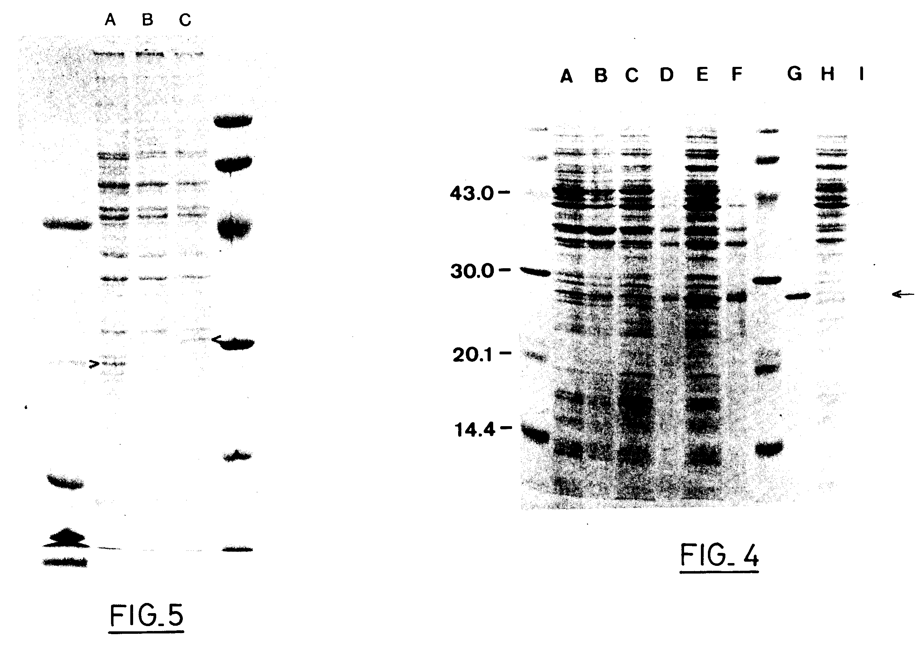

● La figure 5 représente des extraits de S. cerevisiae TGY1sp4 produisant la protéine p28 codée par les constructions pTG1885 et pTG1886, après électrophorèse en gel d'acrylamide-SDS et coloration au bleu de Coomassie.

A. extrait de TGY1sp4/pTG1885 (protéine non fusionnée de 28 kd)

B. extrait de culture témoin TGY1sp4

C. extrait de TGY1sp4/prTG1886 (protéine fusionnée de 30 kd).

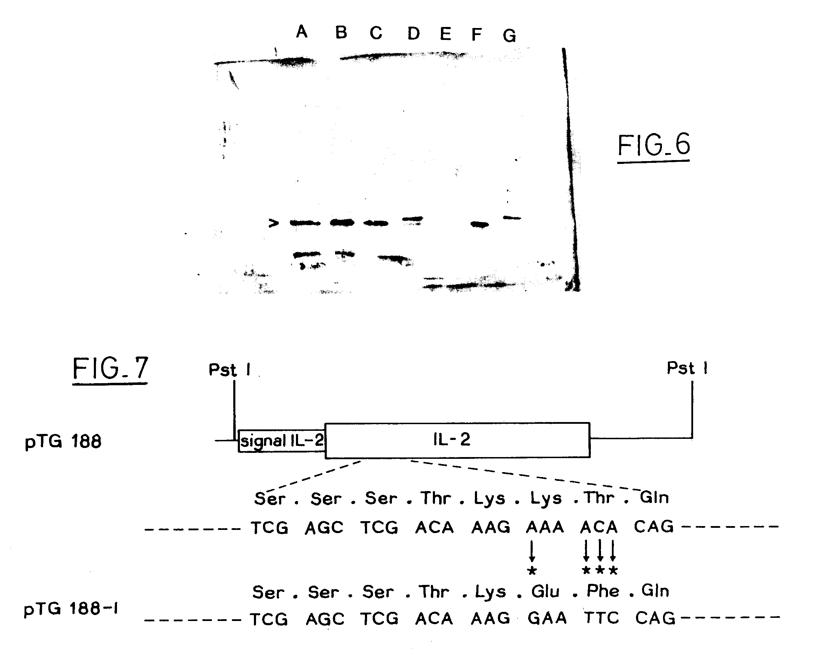

● La figure 6 représente des extraits de E. coli et S. Cerevisiae produisant une protéine de 28 kd fusionnée ou mature, analysés en "Western blot" avec des anticorps de lapin spécifiques de la p28 native. Les anticorps fixés sont reconnus par des anticorps anti-lapin, marqués à la biotine et ce complexe est ensuite révélé par un réactif streptavidine-peroxydase et une coloration par HRP (bio-Rad).

A. extrait total de coli TGE901 pTG44

B. fraction non soluble du même extrait

C. extrait total de S. cerevisiae, TGY1sp4/pTG1885, produisant la protéine mature

D. extrait total de S. cerevisiae produisant la protéine mature fusionnée aux 22 premiers acides aminés de PGK, TGY1sp4/pTG1886

E. extrait de S. cerevisiae témoin négatif

F.G. mêmes extraits que C et D, 2 fois moins concentrés.

● La figure 7 représente la mise en phase du site EcoRI par mutagénèse dirigée dans la région du cADN correspondant aux 9 premiers acides aminés de l'interleukine 2 humaine.

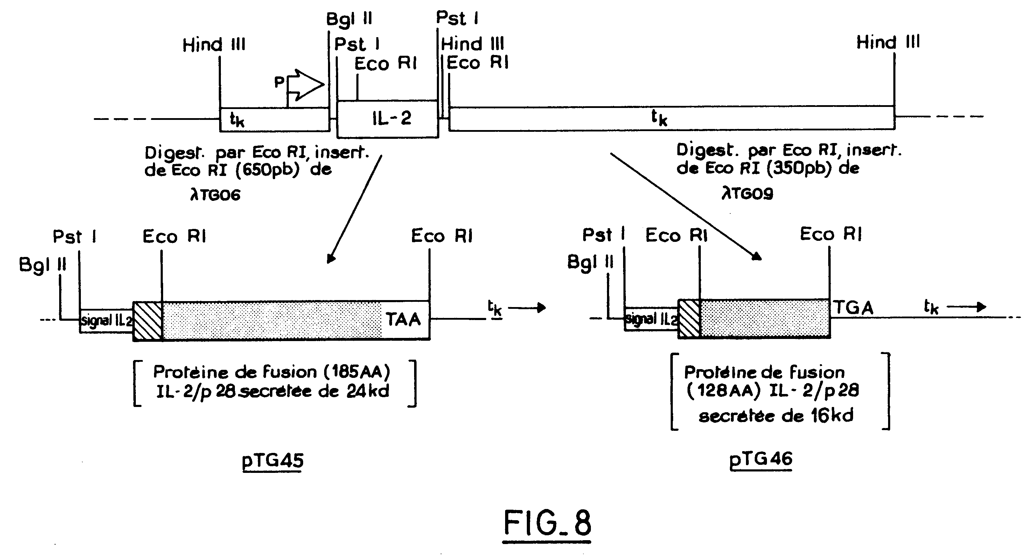

● La figure 8 représente la construction de pTG45 et pTG46 par insertion des fragments EcoRI de λTG06 et λTG09 dans le site EcoRI de pTG188-I (voir construction de la figure 7).

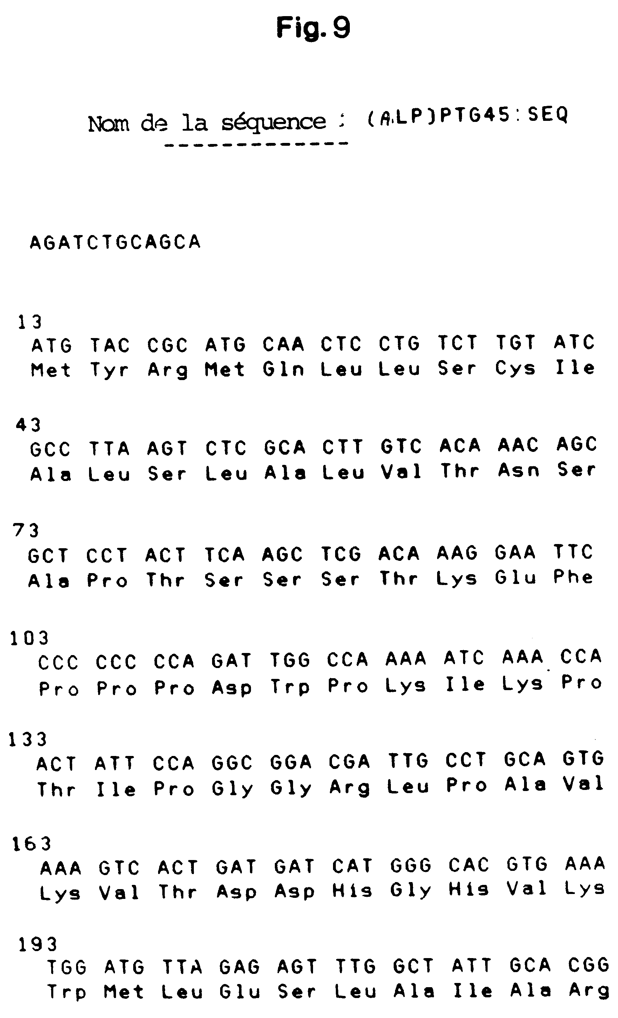

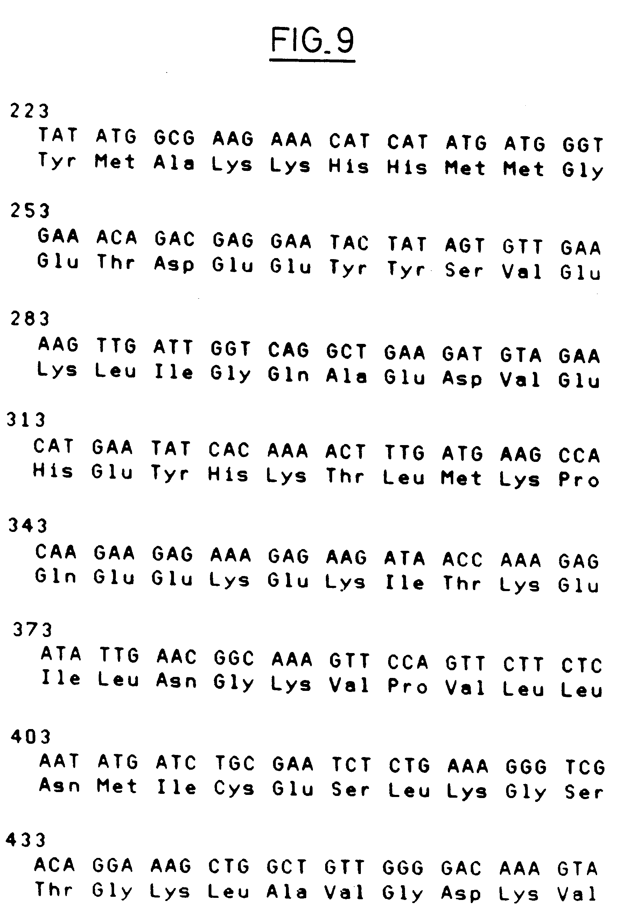

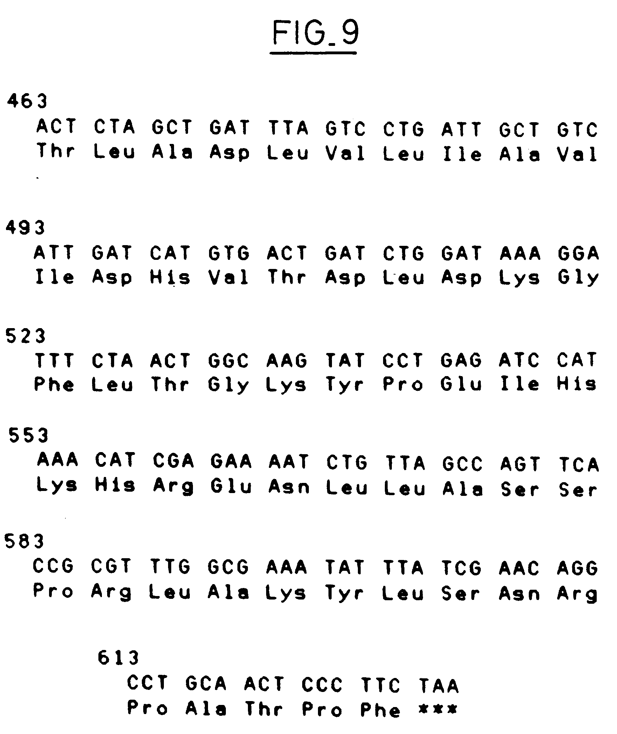

● La figure 9 représente la structure de la protéine de fusion IL2/p28 codée par pTG45. On remarque la présence d'un peptide signal qui permet l'excrétion d'une protéine de 24 kd comprenant la plus grande partie de p28.

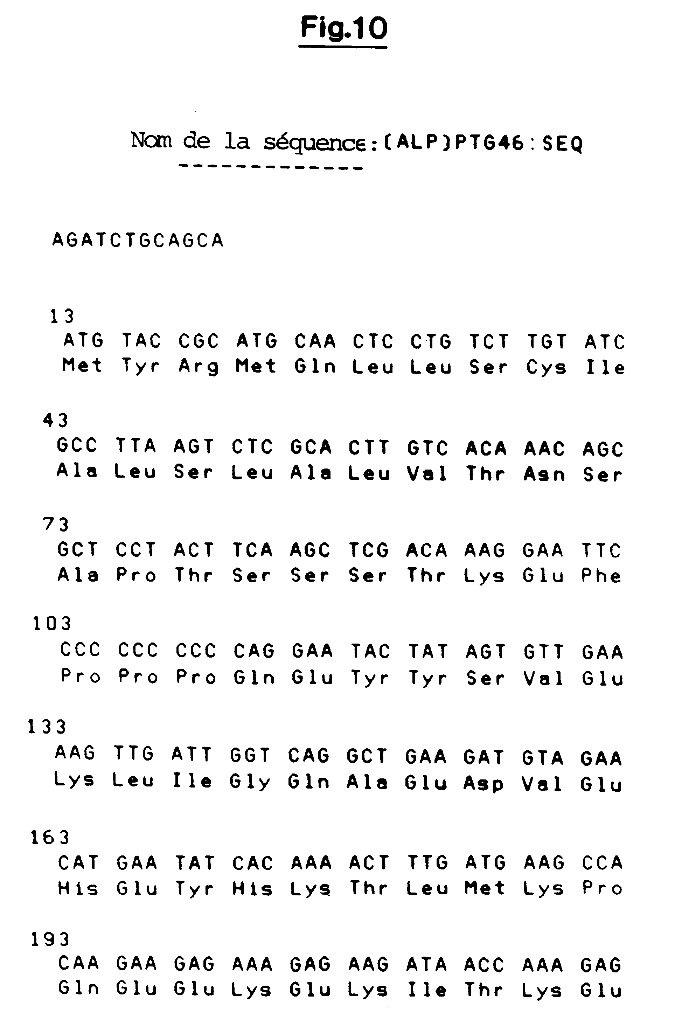

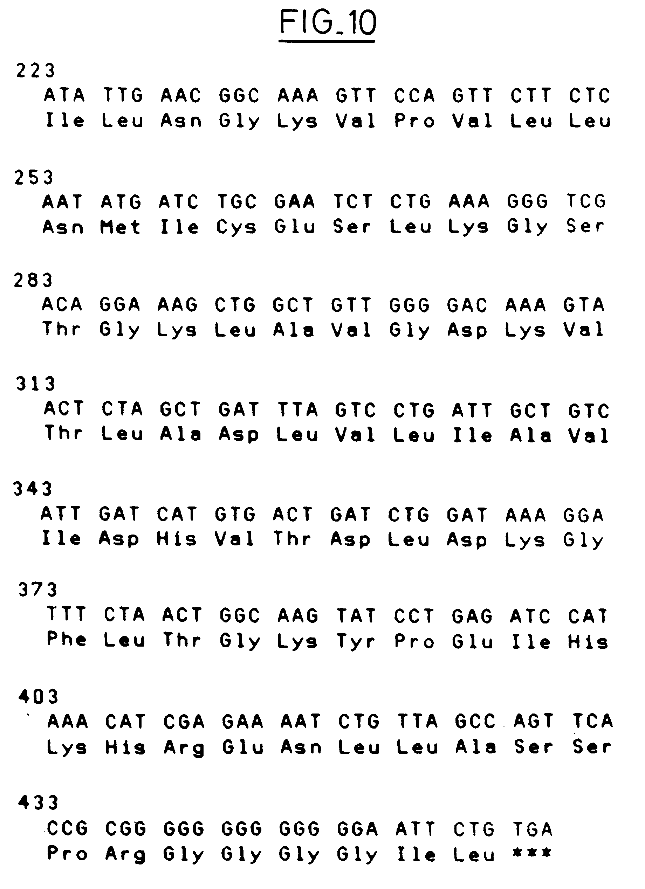

● La figure 10 représente la structure de la protéine de fusion IL2/p28 codée par pTG46. Le peptide signal permet l'excrétion d'une protéine de 16 kd qui contient la partie centrale de l'antigène p28.

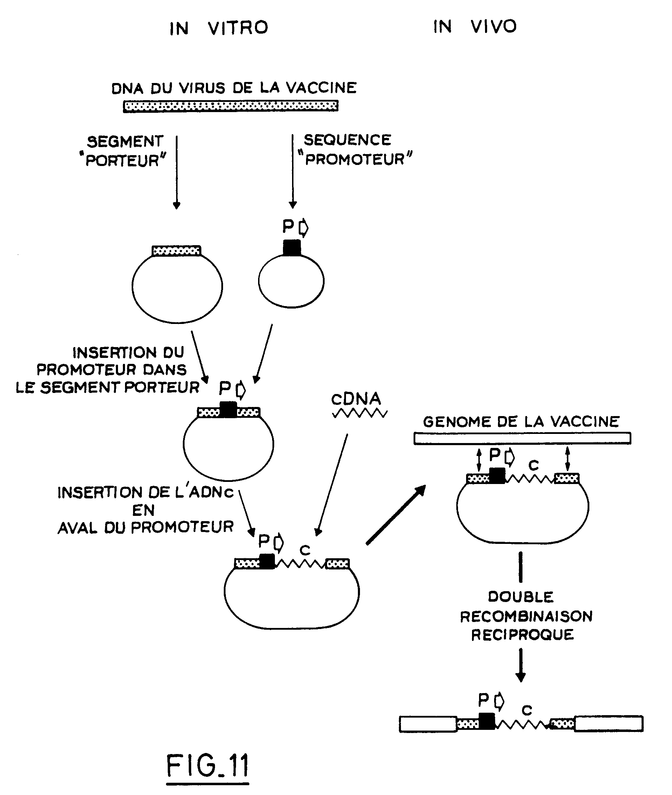

● La figure 11 représente l'insertion dans un plasmide portant le gène vaccinal TK, du gène codant pour IL2/p28 placé sous le contrôle du promoteur vaccinal 7,5 K.

Insertion de ce bloc d'expression dans le génome viral par double recombinaison entre les séquences TK.

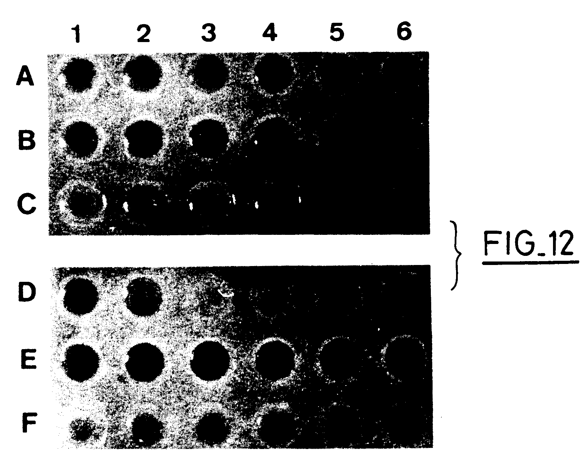

● La figure 12 représente la détection des protéines sécrétées dans le milieu de culture de cellules BHK21 infectées par les virus recombinants VV.TG.p28 Sm-1 et VV.TG.p28 Sm-2, par l'anticorps de lapin ou de rat spécifique de la p28 native

A et D surnageant de VV.TG.p28Sm-1

B et E surnageant de VV.TG.p28Sm-2

C et F surnageant de culture infectée par un VV sauvage

A, B, C détection par des anticorps de lapin

D, E, F détection par des anticorps de rat.

Les numéros correspondent à des isolements indépendants.

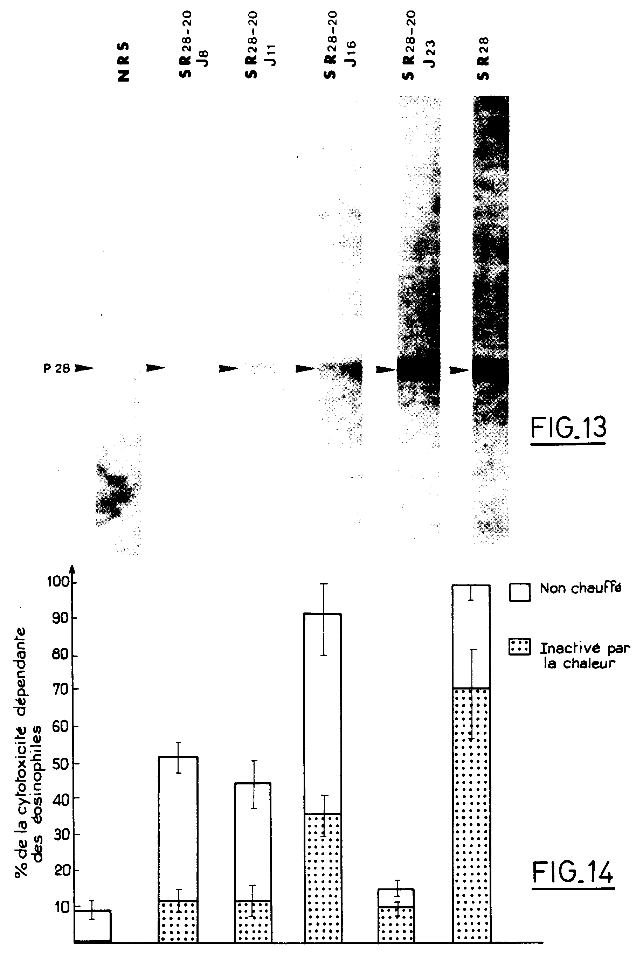

● La figure 13 représente la détection d'anticorps anti-p28 par test "Western blot" avec la protéine p28 native, dans les sérums de rats immunisés avec la protéine cII/ p28 synthétisée par E.coli.

- NRS : sérum de rat contrôle, non inoculé

- SR28-20,J8, J11, J16, J23 : sérum de rats récoltés 8, 11, 16 et 23 jours après l'inoculation de la protéine cII/p28

- SR28 : sérum de rat inoculé avec la protéine p28 native.

● La figure 14 représente l'activité biologique des mêmes sérums que dans la figure 13, mesurée dans un test de cytotoxicité-éosinophile-dépendante contre les schistosomules (voir tableaux I, II et III).

- SR28-20 représente l'antisérum dirigé contre un fragment cloné de 20 kDa correspondant à l'antigène 28 kba de S.mansoni récupéré à différents temps après la seconde injection

- SR-28 représente l'antisérum dirigé contre p28 native et récupéré 14 jours après la seconde injection.

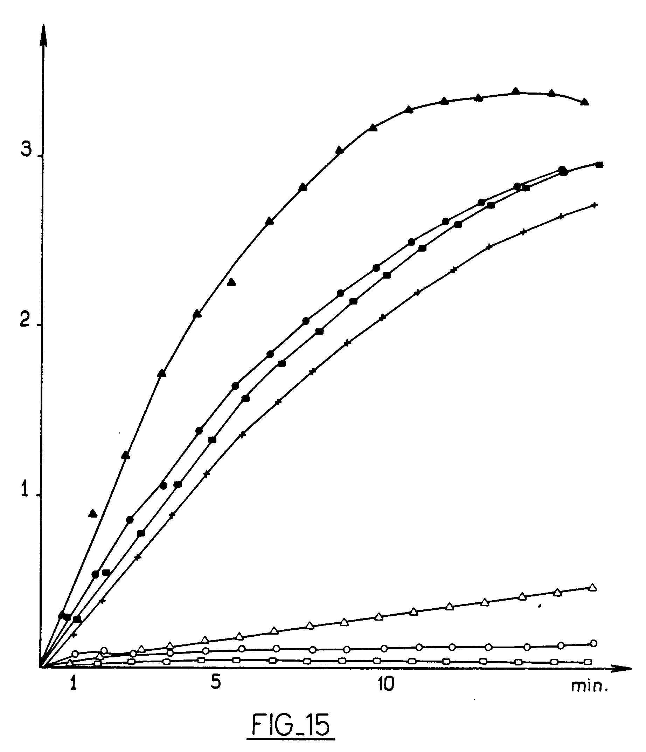

● La figure 15 représente les courbes suivantes :

- GT de foie de rat (▲9 μ g . 6μ g)

- extraits bruts de E.coli, témoin (o) et TGE901/pTG54(●)
- extraits bruts de S. cerevisiae, témoin (■), transformé par un plasmide vecteur (△) et TGY2sp13b/pTG2800 ( + ).

EXEMPLE 1 :

Identification et clonage d'un cADN codant pour une protéine p28.

La stratégie choisie pour cloner le jène codant pour un antigène p28 dont la séquence n'est pas connue a été d'identifier les produits d'expression dans E. coli d'une banque de cADN de schistosomes, à l'aide d'anticorps polyclonaux spécifiquement dressés contre la p28. Ces anticorps ont été induits par immunisation de lapins et de rats avec des extraits du parasite S. mansoni adulte, fractionnés sur gel.

Préparation de la banque de cADN

A partir de l'ARN extrait de schistosomes adultes, on synthétise le brin d'ADN complémentaire par l'action de la transcriptase réverse de MLV. L'ADN rendu double brin par l'action du fragment Klenow de la polymérase de coli est ensuite traité à la nucléase S1 pour rendre ses extrémités franches. On sélectionne sur gradient de sucrose les fragments correspondant à la taille recherchée, c'est-à-dire entre 500 et 4 000 pb. On ajoute à ces fragments une queue de résidus dG et on les insère dans le site EcoRI du dérivé du phage lambda gt 10 (Huynh, 1984), par l'intermédiaire d'un adaptateur synthétique 5'-AATTCCCCCCCCCCC-3' (Le Bouc et al. 1986).

Après ligation, les phages recombinants sont empaquetés in vitro. Une banque de $2.10^6$ phages ainsi constituée est ensuite amplifiée par inoculation dans E. coli POP 101 (Lathe, 1977).

Après amplification, les phages sont concentrés au PEG, purifiés par deux centrifugations en gradient de CICs et leur ADN est extrait. Les inserts de cADN sont récupérés par digestion par EcoRI et purifiés sur gradient de sucrose. Les fractions de 500 à 2 000 pb sont ensuite insérées dans un vecteur d'expression, le dérivé du phage lambda gt 11 qui permet l'expression de gènes étrangers sous le contrôle du promoteur lac, après fusion du cADN avec le gène de la $\beta$-galactosidase de E. coli (Young et Davis, 1983).

Criblage de la banque et sélection de candidats

Un échantillon de cette banque en lambda gt 11 est inoculé sur la souche de E. coli Y1090 (Young et Davis, 1983) à une dilution représentant $1.10^5$ phages par boîte de 90 mm de diamètre.

L'induction de l'expression de la protéine sous contrôle du promoteur lac est déclenchée à 42°C en présence d'isopropyl-$\beta$-D-thiogalactopyranoside.

Les protéines synthétisées sont adsorbées sur filtres de nitrocellulose pour être incubées en présence de l'anticorps de lapin spécifique de p28. Les anticorps fixés sont reconnus par un second anticorps anti-lapin, marqué à la biotine ; ce complexe est ensuite révélé par une réaction à la streptavidine-peroxydase puis une coloration par HRP (Bio Rad).

Cette détection par anticorps permet d'identifier les phages recombinants dont l'insert de cADN dirige la synthèse d'une protéine ou fraction de protéine portant des épitopes reconnus par les anticorps spécifiques anti-p28.

Une première sélection conduit à 3 isolats indépendants, lambda TG06 lambda TG08 et lambda TG09, qui contiennent des inserts de cADN de 650, 700 et 350 paires de bases respectivement.

Les séquences nucléotidiques des inserts portés par ces candidats se chevauchent et comprennent une région qui pourrait correspondre à la région C-terminale d'une protéine hypothétique, d'un poids moléculaire de 28 kd. Un nucléotide synthétique 5'-GGAATAGTTGGTTTGATT-3', marqué au $^{32}$P, complémentaire de l'extrémité 5' de la région codante de l'insert du lambda TG06 a été synthétisé et utilisé pour effectuer un nouveau criblage de la banque de cADN dans lambda gt 10.

De cette façon, deux nouveaux candidats ont pu être isolés, lambda TG10 et lambda TG11 , qui contiennent tous deux une séquence nucléotidique complète codant pour une protéine de 211 acides aminés et correspondant à un PM de 28 kd.

Détermination de la séquence du cADN porté par les phages recombinants et de la protéine correspondante

La séquence complète du cADN (confirmée par la détermination de la séquence des différents candidats) et la structure primaire en acides aminés qui en découle sont présentées dans la figure 1.

Ce résultat et les travaux publiés actuellement ne fournissent aucune information sur la séquence N-terminale du premier produit de traduction du gène natif ni sur l'existence éventuelle d'un précurseur et d'un peptide signal précédant la protéine mature.

Toutefois, la localisation exacte du premier aminoacide n'est pas essentielle dans le contexte de l'invention car la stratégie adoptée pour l'isolement de ce gène et pour son expression comprend la recherche des épitopes majeurs qui sont présents dans la p28 et qui sont reconnus par le système immunitaire de l'hôte.

Cet argument sera utilisé dans les exemples suivants qui décrivent l'expression des épitopes importants dans différents organismes.

Expression de l'antigène p28

La stratégie générale développée pour l'expression des épitopes antigéniques présents dans la p28 a été de fusionner le fragment cloné de cADN dans la phase de lecture correcte à une séquence nucléotidique codant pour la région N-terminale d'une protéine efficacement exprimée et bien caractérisée. La protéine hybride obtenue ainsi sera en général reconnue par les anticorps dressés contre la p28 mature native et on peut espérer qu'elle induise une réponse immunitaire contre le parasite. Avec cette hypothèse de travail différents systèmes d'hôtes ont été utilisés pour analyser les propriétés immunogéniques des protéines de fusion, à la fois in vivo et in vitro, et seront développés dans les exemples suivants.

EXEMPLE 2 :

Expression dans E. coli

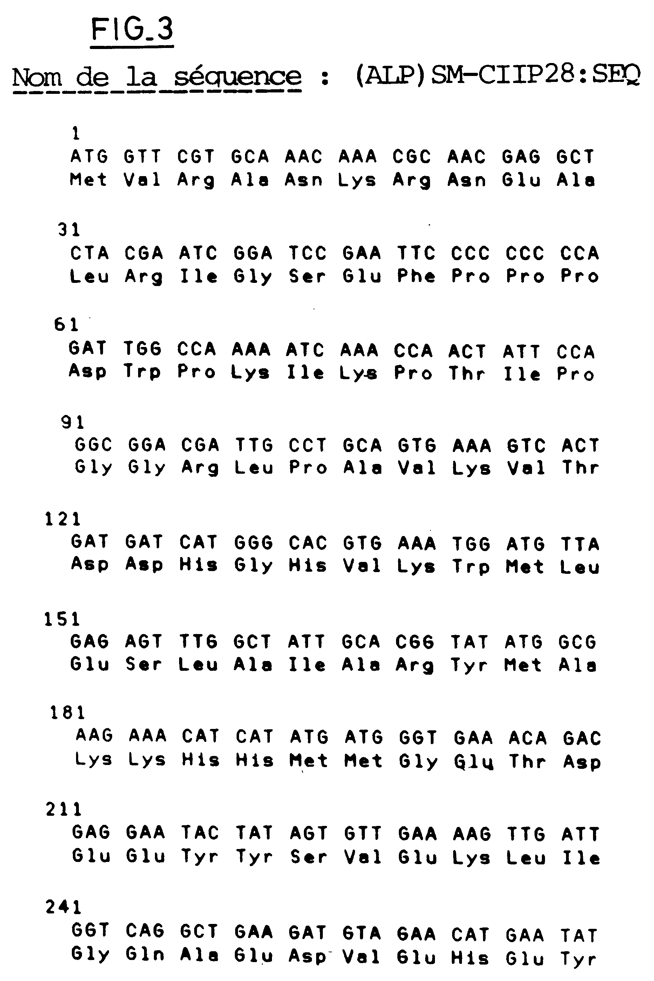

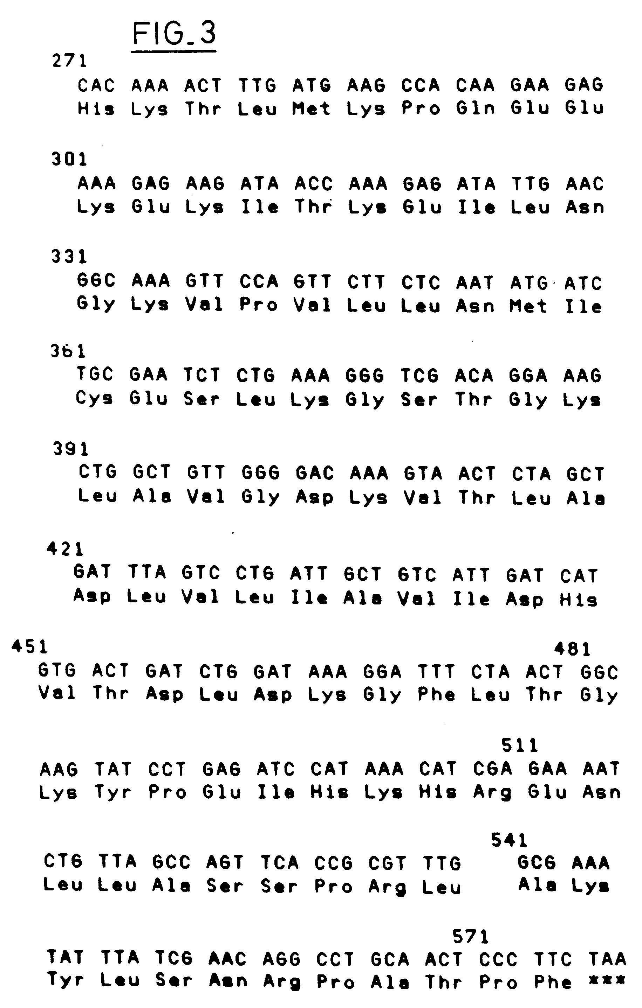

Pour obtenir l'expression d'un antigène p28 dans E. coli, un dérivé du plasmide d'expression pTG908, décrit dans la demande de brevet 83 00909, a été utilisé pour synthétiser une protéine de fusion dont la structure est indiquée dans la figure 3.

Le vecteur pTG908 contient une origine de réplication, le promoteur $P_L$ du bactériophage lambda et la séquence de fixation des ribosomes de cII incluant l'extrémité N-terminale du gène cII avec des sites de restriction appropriés en aval du signal d'initiation de la traduction. Le site BamHI à 39 pb en aval du codon ATG a été utilisé pour insérer un site EcoRI au moyen d'un adaptateur double brin comprenant les oligomères suivants :
5'-dGATCCGAATTCG-3'
3'-GCTTAAGCCTAGd-5'

Le plasmide résultant, pTG1924, contient un site EcoRI unique avec le codon GAA en phase avec le codon d'initiation du gène cII. Tous les inserts de cADN provenant des recombinants lambda gt11 qui ont donné un signal positif après sélection avec le sérum anti-p28 ont été insérés directement dans ce site. Quand l'insert se retrouve dans l'orientation correcte, ceci donne une séquence de nucléotides codant pour l'extrémité N-terminale du gène cII et continuant en phase avec la séquence de cADN codant pour l'antigène p28 ou pour une partie de celui-ci.

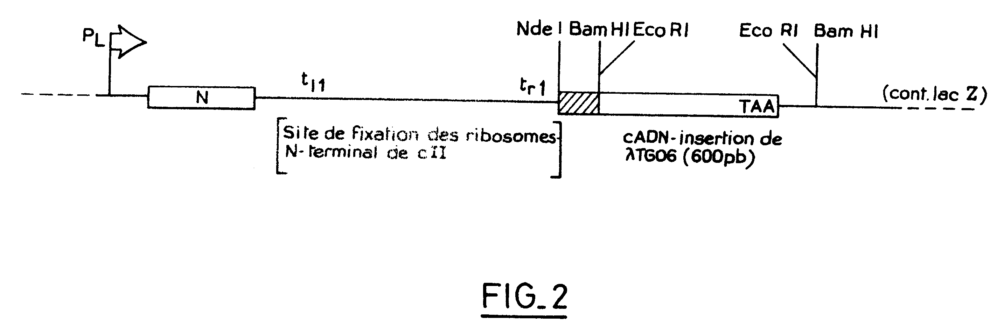

Une des constructions plasmidiques résultantes, pTG44, contenant l'insertion dans ce site EcoRI du fragment dérivé de lambda TG 06 est représentée à la figure 2. La séquence de la protéine de fusion cII-p28 avec un poids moléculaire calculé de 25 kd est donnée à la figure 3.

Une culture de E. coli N4830 transformée par pTG44 est mise en croissance sur milieu LB jusqu'à une $DO_{600}$ de 0,2 puis la synthèse de la protéine de fusion est induite par une augmentation de température à 42°C, pendant les 8 heures suivantes.

En fin de culture, les extraits cellulaires ont été analysés sur gel d'acrylamide-SDS par coloration au bleu de Coomassie (figure 4). Le produit de fusion cII-p28 est récupéré dans la fraction non soluble obtenue après lyse des cellules par sonication. La protéine est récupérée avec une pureté de l'ordre de 80 % par extraction de la fraction non soluble en présence de 0,2 % de SDS. Une analyse par "Western blot" de la préparation finale montre que cette protéine particulière est efficacement reconnue par les anticorps de lapin dressés contre l'antigène de la p28 native (figure 5).

EXEMPLE 3

EXPRESSION DANS LA LEVURE S. CEREVISIAE

Deux types de constructions ont été réalisées pour exprimer le cADN de p28 dans la levure : la première aboutit à une protéine de fusion comprenant, du côté N-terminal, les 22 premiers acides aminés de la phosphoglycérate kinase (PGK) de levure ; la seconde donne une protéine semblable à la protéine mature naturelle.

- CONSTRUCTION D'UN VECTEUR PORTANT UNE FUSION DE GENES CODANT POUR PGK ET p28 (pTG 1886)

1°) L'insert EcoRI du lambda TG10 contenant le cADN de la protéine p28 a été introduit dans un plasmide navette coli-levure, pTG 836.
  • ce plasmide est un dérivé de pBR322 qui contient l'origine de réplication du plasmide de levure 2 microns, le gène URA 3 (brevet 84.12598) et le gène PGK de levure (Hitzemann et al. 1982).
  • un site unique EcoRI a été introduit entre le site BamHI (situé 20 codons en aval du codon initiateur de PGK) et le site Sal1 (présent dans la séquence du pBR322), par insertion d'un adaptateur synthétique :
    5'. GATCTGAATTCAGATCTC- 3'
    3' - ACTTAAGTCTAGAGAGCT- 5'
    le plasmide résultant est le pTG 1880
  • l'insertion du fragment EcoRI de lambda TG10 dans le site EcoRI de pTG 1880, dans l'orientation correcte donne le pTG 1883.
2°) Le bloc d'expression PGK-p28 porté par ce plasmide va être repris pour être inséré dans un vecteur d'expression de levure, le pTG 848 (brevet français 85.06672). Ceci nécessite une manipulation intermédiaire dans le phage M13 :
  • un fragment HindIII-BglII contenant le bloc d'expression PGK-p28 est inséré dans le M13TG131 (Kieny et al. 1983) entre les sites HindIII et BamHI, pour donner le M13TG1884.
  • cette construction permet, par l'existence de sites de restriction voisins, de récupérer le même bloc d'expression sous forme de fragment SmaI-BglII.
  • ce fragment est ensuite introduit entre les sites SmaI et BglII du vecteur d'expression pTG848.
  • le plasmide résultant est le pTG1886.
3 ) La levure TGY1sp4 a été transformée avec ce plasmide pTG1886.
Des extraits cellulaires bruts de ces cultures ont été analysés par électrophorèse sur gel d'acrylamide - SDS et par la technique de "Western blot" Les figures 5 et 6 montrent clairement l'apparition d'une nouvelle bande protéique d'un PM de 30 kd, comme attendu pour la protéine de fusion PGK-p28 et qui est reconnue par les anticorps dressés contre la p28 native extraite des schistosomes.

- CONSTRUCTION D'UN VECTEUR D'EXPRESSION PORTANT SEULEMENT LE cADN DE p28, SOUS LE CONTROLE DU PROMOTEUR PGK

1°) Dans la construction M13TG1884 décrite plus haut, le codon initiateur de la protéine p28 est précédé des 22 premiers codons du gène PGK et de 8 codons dus à la technique de clonage (en particulier les résidus dC). Ces 90 paires de bases additionnelles ont été délétées par mutagénèse in vitro du M13TG1884 simple brin en présence de l'oligonucléotide :

$$5'\text{-CAACAAAATATAAACA}\boxed{\text{ATG}}\text{CTGGCGAGCATATC-}3'$$

qui correspond aux 16 premiers nucléotides de la séquence leader du mRNA du PGK suivi des nucléotides du début de la séquence codante de p28 ; entre ces 2 séquences se trouve l'ATG qui coïncide à la fois avec l'ATG initiateur du PGK et celui de p28.
2°) Le dérivé correctement délété, M13TG1884m a été digéré par SmaI et BlgII et le fragment portant le cADN de p28 non fusionné, placé sous le contrôle du promoteur de PGK, a été inséré dans le vecteur d'expression pTG848 (comme décrit plus haut), pour donner le pTG1885.

3°) La levure TGY1sp4 a été transformée avec ce plasmide pTG1885.

Des extraits cellulaires bruts de ces cultures ont été analysés par électrophorèse sur gel d'acrylamide-SDS et par la technique de "Western blot". Les figures 5 et 6 montrent clairement l'apparition d'une nouvelle bande protéique d'un PM de 28 kd et qui est reconnue par les anticorps dressés contre la p28 native extraite des schistosomes.

EXEMPLE 4

EXPRESSION DANS DES VIRUS DE LA VACCINE RECOMBINANTS

a) Construction d'un virus de la vaccine recombinant exprimant une p28 fusionnée aux premiers acides aminés de l'IL-2.

Les cADN portés par les phages lambda TG06 et TG09 ont été introduits dans des vecteurs appropriés pour pouvoir ultérieurement être insérés dans le génome du virus de la vaccine et, ainsi, être exprimés dans des cellules de mammifères.

Les vecteurs selon la présente invention dérivent des vecteurs qui ont été mis en oeuvre pour l'expression de l'interleukine-2-humaine (voir brevet FR-N° 85 09480 ). On a ainsi construit un dérivé de pTG188 qui contient un site EcoRI dans une région de la séquence nucléotidique qui correspond à 9 amino-acides en aval du résidu alanine N-terminal de l'interleukine-2 native (figure 7). Les inserts de cADN de lambda TG06 et lambda TG09 ont été insérés en orientation correcte entre les sites EcoRI de pTG188-I pour donner les plasmides pTG45 et pTG46 qui codent pour une protéine de fusion IL-2/p28 d'un poids moléculaire de 24 kd et 16 kd respectivement (figure 8), comme cela est représenté figures 9 et 10. La présence d'un peptide signal en amont de la construction permet la sécrétion de la protéine dans le milieu de culture.

Les parties importantes des plasmides pTG45 et pTG46 comportent le gène viral TK interrompu par la séquence codant pour la protéine de fusion $IL_2$-p28, placée sous le contrôle d'un promoteur efficace, celui de la protéine vaccinale 7,5K.

Ces séquences insérées dans le gène TK de la vaccine peuvent être transférées dans le génome du virus de la vaccine par une double recombinaison réciproque (figure 11) comme cela a été décrit précédemment (Panicalli et Paoletti, 1982 ; Mackett et al., 1982 ; Kieny et al., 1984).

Les virus recombinants VV.TG.p28Sm-1 et VV.TG.p28Sm-2 contenant les cADN dérivés de lambda TG06 et lambda TG09 respectivement sont utilisés pour infecter une monocouche de cellules BHK21. Après 24 heures à 37°C, le surnageant de la culture est testé pour déterminer la présence de la protéine de fusion IL-2/p28, ceci par adsorption sur filtre de nitrocellulose et incubation avec des anticorps de rat ou de lapin, spécifiques de la p28 native.

La détection des anticorps fixés est effectuée par incubation avec un antisérum biotinylé dressé contre les immunoglobulines totales de rat ou de lapin ; ce complexe est ensuite révélé par une réaction à la streptavidine-peroxydase et une coloration finale en présence d'un réactif colorant HRP (Bio-Rad).

Aussi bien pour VV.TG.p28Sm-1 que VV.TG.p28Sm-2, des antigènes spécifiques (plus de 100 ng/ml) peuvent être détectés (figure 12) dans le milieu de culture et ceci pour différents recombinants indépendants, alors que les surnageants de cellules infectées uniquement avec le virus sauvage ne donnent pas de signal significatif.

b) Construction d'un virus de la vaccine recombinant exprimant une p28 fusionnée au peptide signal de la glycoprotéine rabique (VV.TG.1184)

- Construction du bactériophage M13TG177

Le bactériophage M13TG169 (brevet FR 86.05043) contient la séquence codante du gène env du virus HIV-1 flanquée à l'extrémité 5' de la séquence codant pour le peptide signal de la glycoprotéine rabique) et à l'extrémité 3' de la séquence codant pour la zone transmembranaire et la zone intracytoplasmique de la glycoprotéine rabique. Un site EcoRI a été introduit entre les sites KpnI et HindIII de M13TG169 pour générer M13TG176, de structure suivante :

S : signal de la gp rabique
TM : zone transmembranaire de la gp rabique

Le site EcoRI situé en aval du peptide signal a ensuite été éliminé par mutagénèse localisée à l'aide de l'oligonucléotide suivant :

5' GGGGAAATCGTAATC 3'

Le bactériophage M13TG177 résultant possède donc un site EcoRI unique.

- Construction du bactériophage M13TG1105

Le fragment de restriction EcoRI de λTG10 contenant la séquence codante de la p28 est introduit dans M13TG177 pour générer
M13TG1105, de structure suivante :

- Construction du bactériophage M13TG1108

Les deux premiers acides aminés suivant le peptide signal S sont fusionnés en phase avec la séquence codante de la p28 par mutagénèse localisée avec l'oligonucléotide suivant :

5'CTTGATATGCTCGCCAGCAATAGGGAATTTCCCAAA 3'

Le bactériophage résultant est appelé M13TG1108.

- Construction du plasmide pTG1184

Le bactériophage M13TG1108 est digéré partiellement par PstI afin d'isoler le fragment contenant la totalité de la séquence codant pour la p28 et inséré au site PstI du plasmide pTG186POLY pour générer pTG1184.

c) Construction d'un virus de la vaccine recombinant exprimant une p28 fusionnée au peptide signal et à la zone transmembranaire de la glycoprotéine rabique (VV.TG.1185)

- Construction du bactériophage M13TG1109

Dans le bactériophage M13TG1108, le dernier acide aminé avant le codon de terminaison de traduction est fusionné en phase avec le premier acide aminé de la zone transmembranaire TM grâce à l'oligonucléotide suivant :

5'TGCACTCAGTAATACATAGAAGGGAGTTGCAGGCCT3'

Le bactériophage résultant est appelé M13TG1109.

- Construction du plasmide pTG1185

Comme pour la construction de pTG1184, le bactériophage M13TG1109 est soumis à digestion partielle par PstI et le fragment contenant la totalité de la séquence codant pour la p28 est inséré au site PstI de pTG186POLY pour générer pTG1185.

d) Caractérisation des virus VV.TG.1184 et 1185

Les plasmides pTG1184 et 1185 sont utilisés pour transférer le gène codant pour la p28 dans le génome du virus de la vaccine comme décrit précédemment.

Le virus VV.TG.1184 exprime la p28 fusionnée au peptide signal de la glycoprotéine rabique.

Le virus VV.TG.1185 exprime la p28 fusionnée au peptide signal et à la zone transmembranaire de la glycoprotéine rabique.

Des cellules BHK21 sont infectées avec les virus VV.TG.1184 ou 1185 (0,2 ufp/cellule) pendant 16 heures. Après ajout de méthionine $^{35}$S pendant 4 h, les protéines marquées sont immuno-précipitées à l'aide d'un anticorps de lapin dirigé contre la p28. On met en évidence dans le cas du virus VV.TG.1184 une bande correspondant à un poids moléculaire de 28 kd. Cette protéine est également présente de façon abondante dans le surnageant de culture, montrant que la protéine est sécrétée.

Dans le cas du virus VV.TG.1185, on met en évidence une bande correspondant à un poids moléculaire de 35 kd. Cette protéine est absente des surnageants de culture, démontrant qu'elle est retenue dans la membrane cellulaire par la zone transmembranaire de la glycoprotéine rabique.

EXEMPLE 5

VACCINATION DES ANIMAUX AVEC L'ANTIGENE p28 PRODUIT PAR MANIPULATION GENETIQUE

L'effet biologique des protéines recombinantes contenant un ou plusieurs épitopes majeurs de p28 native a été analysé en comparaison avec la réponse observée après immunisation de rats avec la p28 native mature isolée d'extraits totaux de parasites adultes. Les protéines de fusion produites à la fois par les microorganismes tels que E. coli ou S. cerevisiae ou par un virus de la vaccine recombinant peuvent être utilisées pour une telle approche, mais seuls les résultats obtenus avec la protéine de fusion cII/p28 sont explicités ci-après.

- IMMUNISATION DES RATS

20 rats mâles "Fisher" âgés de 3 mois sont immunisés par injection intrapéritonéale avec 50 $\mu$g d'une protéine de fusion cII/p28 (exemple 2) en présence d'adjuvant de Freund. Les animaux reçoivent une seconde dose après 2 semaines et les antisérums sont prélevés à partir du jour 8.

4 "pools" de sérums comprenant 5 échantillons chacun sont essayés en 3 fois pour tester la présence d'anticorps reconnaissant la protéine p28 native et la cytotoxicité des sérums à l'égard des schistosomules.

- DETECTION DES ANTICORPS SPECIFIQUES

Les sérums des rats immunisés avec la protéine cII/p28 extraite de E. coli sont évalués dans un test de "Western-blot" pour leur réaction contre la p28 d'un extrait total de parasites adultes, séparée par électrophorèse sur gel de polyacrylamide-SDS. La figure 13 montre une reconnaissance non ambiguë de la protéine p28 native par ces sérums.

L'intensité maximale de cette réponse est atteinte au jour 23 et est pratiquement égale à celle observée avec les sérums d'animaux immunisés avec l'antigène natif.

- EVALUATION DE LA CYTOTOXICITE DEPENDANTE DES EOSINOPHILES

Les mêmes sérums ont été évalués dans un test de cytotoxicité dépendante des éosinophiles, selon la technique décrite par Capron et al. (1981) :

50 schistosomules de S. mansoni sont incubés en présence de chaque sérum à tester (non chauffé), ou des contrôles appropriés, et en présence d'éosinophiles (cellules péritonéales non adhérentes de rat Lou contenant de 40 à 70 % d'eosinophiles). Le mélange réactionnel comprend 6000 cellules effectrices pour 1 schistosomule, dans un volume total de 200 $\mu$1. Après 48 heures d'incubation à 37°C, le pourcentage de mortalité des schistosomules est évalué par examen au microscope.

Les résultats présentés dans le Tableau I montrent clairement la présence d'un facteur cytotoxique capable d'induire un haut taux de mortalité des schistosomules, taux très proche de celui induit par un sérum de rat infecté par S. mansoni.

## - MISE EN EVIDENCE DU ROLE D'IgE SPECIFIQUES DANS LA REACTION DE CYTOTOXICITE

- Le même test de cytotoxicité a été effectué avec du sérum chauffé (2 heures à 56°C) et avec du sérum chauffé additionné de complément. Les résultats présentés dans le Tableau II montrent que le facteur responsable de la cytotoxicité est thermosensible et que cette perte d'activité ne peut pas être compensée par l'addition de complément.
- De plus, on peut éliminer sélectivement les IgE des sérums à étudier par une adsorption par un antisérum de chèvre spécifique des IgE de rat, couplé à du sépharose B. Les sérums ainsi traités, puis dialysés, sont testés pour leur activité cytotoxique dépendante des éosinophiles.

Le Tableau III montre que la déplétion sélective des sérums de rats en IgE empêche l'expression de la cytotoxicité. Celle-ci est donc bien spécifique des IgE des sérums de rats immunisés par la protéine cII/p28.

## MISE EN EVIDENCE DE LA PROTECTION DES ANIMAUX CONTRE UNE INJECTION D'EPREUVE PAR DES CERCAIRES

Des rats Fischer ont été injectés (comme décrit plus haut) avec l'antigène p28 produit dans E. coli ou dans la levure, en présence d'hydroxyde d'aluminium, ou avec du virus de la vaccine exprimant l'antigène p28, VV.TG.1185 ($5.10^7$ ufp de virus recombinant par animal).

Les animaux témoins sont injectés avec de l'hydroxyde d'aluminium seul.

Six semaines après l'immunisation, les animaux sont infectés par voie sous-cutanée avec 1.000 cercaires (état larvaire infectieux du schistosome).

21 jours après l'inoculation d'épreuve, les rats sont sacrifiés et on évalue leur contenu en parasites adultes. Ceux-ci sont récoltés par perfusion de la veine porte-hépatique avec de l'eau physiologique, puis comptés.

On observe une réduction de la charge parasitaire par rapport aux témoins de

64 ± 4 % avec l'antigène produit dans E. coli

70 ± 10 % avec l'antigène produit dans la levure

60 ± 8 % avec l'antigène produit par la vaccine.

**Tableau I**

**Test de cytotoxicité dépendante d'éosinophiles**

**Etude des sérums de rats immunisés par la protéine cII/p28 produite dans E. coli.**

| Source d'anticorps | Dilution finale du sérum | % de cytotoxicité après 48 heures d'incubation |
|---|---|---|
| **Sérum de rat immunisé par cII/p28, récolté au jour** | | |
| J8 | 1/16 | 60.5 ± 2.5 |
| | 1/32 | 55.5 ± 5 |
| | 1/64 | 6.5 ± 6.5 |
| J10 | 1/16 | 85.5 ± 0.5 |
| | 1/32 | 76 ± 3.5 |
| | 1/64 | 57 ± 4.7 |
| J16 | 1/16 | 85.5 ± 0.5 |
| | 1/32 | 78 ± 6 |
| | 1/64 | 52 ± 3 |
| J23 | 1/16 | 88 ± 3.5 |
| | 1/32 | 92 ± 0 |
| | 1/64 | 52 ± 11.5 |
| **Sérum de rat infecté par S. mansoni** | 1/16 | 97 ± 1 |
| **Sérum de rat sain** | 1/16 | 0 |

Tableau II

| Cytotoxicité dépendante d'éosinophiles Etude de la participation du complément | |
|---|---|
| Sérum de rat immunisé par la protéine cII/p28 produite par E. coli (dilution finale : 1/16) 16 jours après immunisation | % de cytotoxicité après 48 heures d'incubation |
| Sérum non chauffé | 85.5 ± 0.5 |
| Sérum chauffé | 26.5 ± 5.5 |
| Sérum chauffé + complément de cobaye | 38.5 ± 0.5 |
| Sérum chauffé + complément de cobaye chauffé | 21.5 ± 1.5 |

## Tableau III

### Cytotoxicité dépendante d'éosinophiles

### Etude de la participation des anticorps d'isotype IgE

| Source d'anticorps | % de cytotoxicité après 48 heures d'incubation |
|---|---|
| **Sérum de rat immunisé par cII/p28 produite dans E. coli, récolté au jour** | |
| J16 | 48.5 |
| J23 | 40 |
| **Sérum de rat sain** | 0 |
| **Effluent de colonne anti-IgE** | |
| J16 | 0 |
| J23 | 0 |
| **Sérum de rat sain** | 0 |

Exemple 6

L'activité glutathion-S-transférase a été mise en évidence dans les extraits de E. coli TGE901/pTG54 et de S. cerevisiae TGY2spl3b/pTG2800 exprimant la protéine P28 de Schistosoma mansoni (pTG2800 ne diffère du pTG1894 décrit dans l'exemple 3 que par une délétion de 170 pb dans la région promoteur du gène ura3).

Les cultures sont centrifugées et les culots sont remis en suspension dans du tampon (100 mM Tris-HCl pH 7.5, 1 mM DTT). Les levures sont broyées avec des billes de verre ; les bactéries sont traitées aux ultra-sons. Les extraits sont centrifugés pour éliminer les débris cellulaires et l'activité est mesurée dans le surnageant (selon les techniques publiées par Moore et al. 1986 et Habig et al. 1974). On mélange, dans une cuvette à spectrophotomètre, de 40 à 100 $\mu$1 d'extrait brut, 10 $\mu$1 de réactif CDNB 100 mM (1 chloro-2,4 dinitrobenzène en solution dans l'éthanol 100 %) et 1 ml de $KH_2PO_4$ 100 mM (pH 6.5)/glutathion réduit 2,5 mM.

On a utilisé, comme témoin positif, de la glutathion-S-transférase de rat (fournie par Sigma) à une concentration finale de 9 $\mu$g et 6 $\mu$g par test.

On a utilisé, comme témoins négatifs, un extrait de E. coli TGE901/pTG959 portant un vecteur sans séquence P28 et un extrait de S. cerevisiae TGY2spl3b et TGY2spl3b/pTG848 portant un vecteur sans séquence P28.

Tous les extraits de bactéries et de levures sont ajustés à une concentration en protéines totales de 0,33 mg/ml.

Une couleur jaune apparaît dans les cuvettes où existe une activité glutathion-transférase. Le changement de D.O. est mesuré au spectrophotomètre à 340 nm, toutes les minutes pendant 15 minutes.

Les résultats présentés dans la figure 15 montrent que les extraits de E. coli et S. cerevisiae qui contiennent la protéine p28I recombinante présentent une forte activité glutathion-S-transférase.

Exemple 7 Criblage de la banque et sélection du candidat $\lambda$TG07.

Le criblage de la banque de cADN en $\lambda$gtll, décrite dans l'exemple 1 a permis de révéler plusieurs types de candidats.

Par immunodétection avec un anticorps polyclonal de rat (induit par injection de la P28 purifiée à partir de schistosomes) on a sélectionné un nouveau candidat, $\lambda$TG07, qui n'est pas reconnu par les sondes synthétiques qui ont été utilisées pour sélectionner les candidats $\lambda$TG10 et $\lambda$TG11, décrits dans l'exemple 1 et dont la séquence est donc différente. Comme la protéine est reconnue par les mêmes anticorps anti-P28, cette nouvelle protéine sera appelée P28II (et celle qui avait été identifiée précédemment sera appelée P28I).

Exemple 8 Détermination de la séquence du cADN porté par le phage $\lambda$TG07.

Divers fragments de restriction du cADN ont été reclonés dans M13 pour être séquencés.

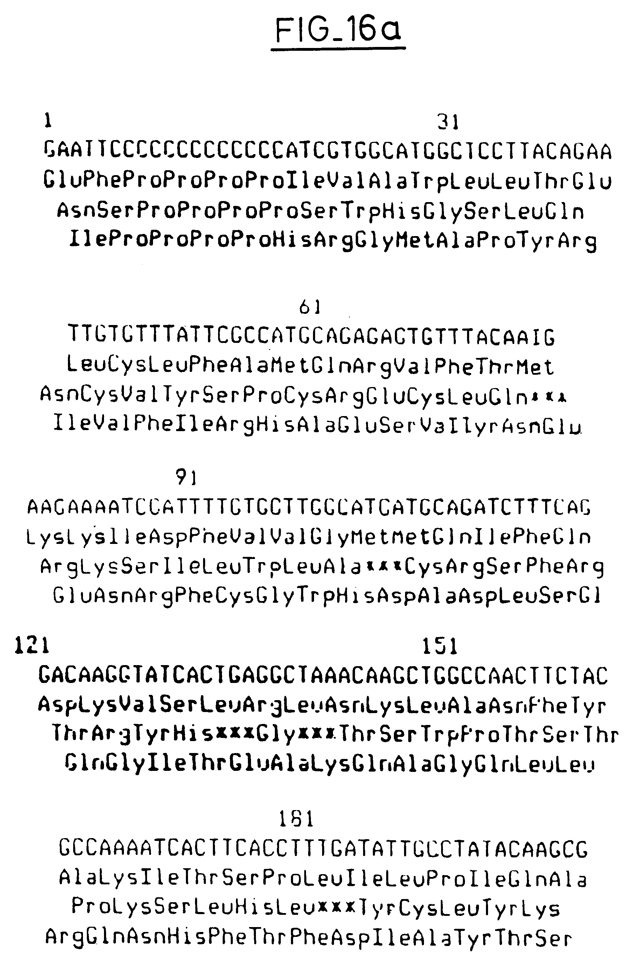

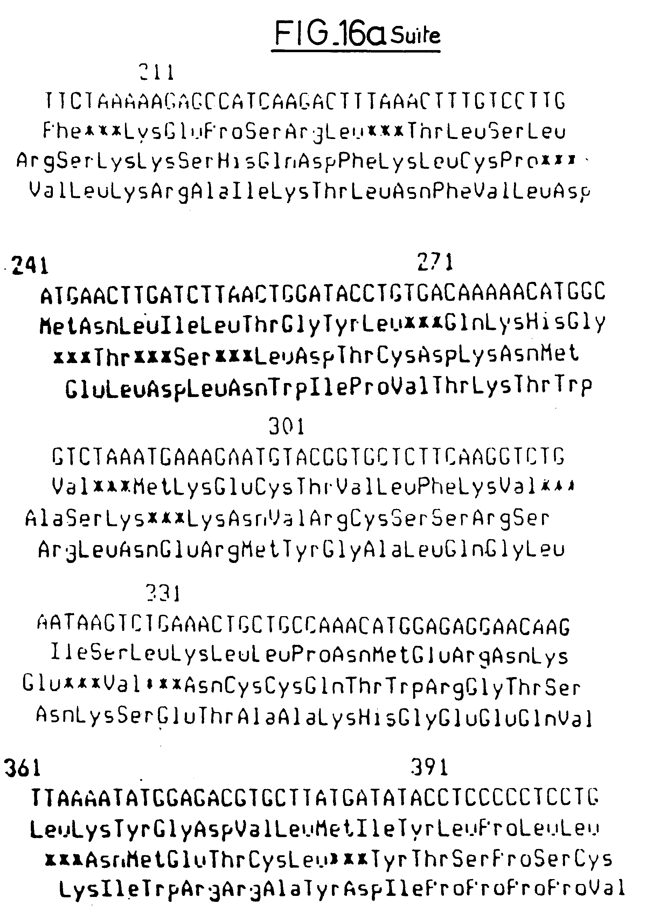

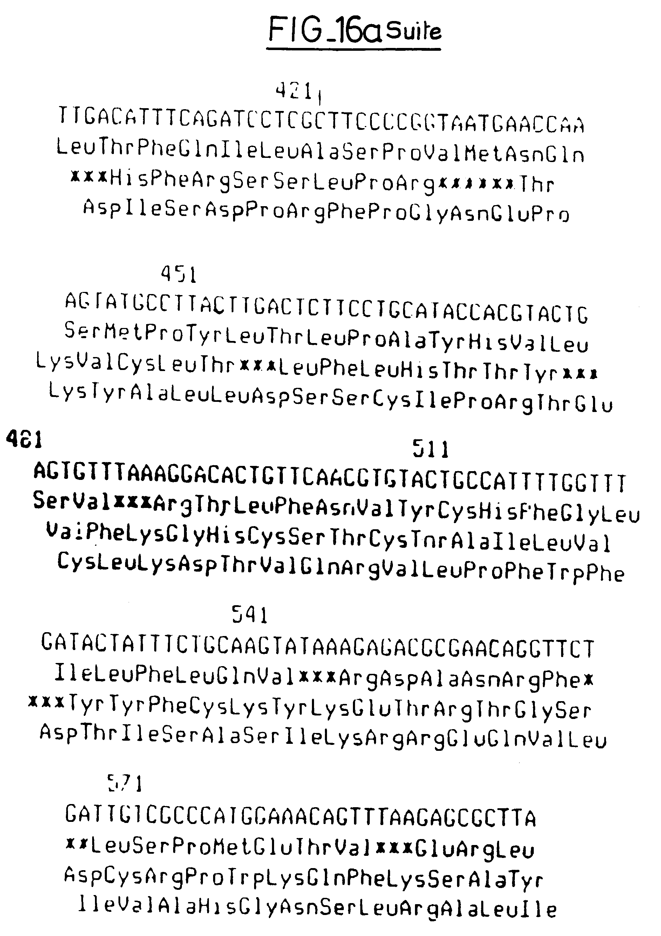

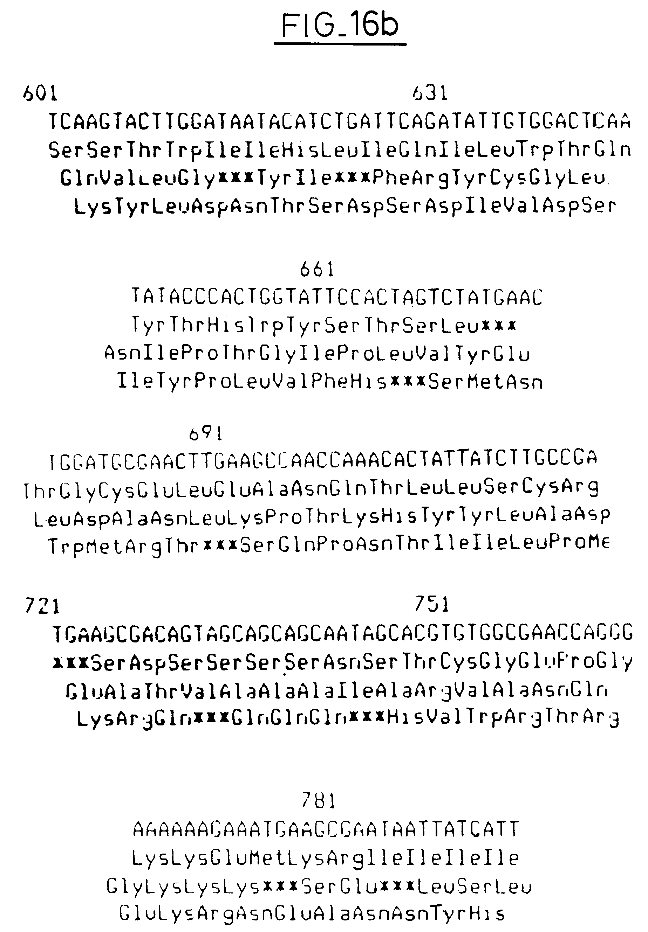

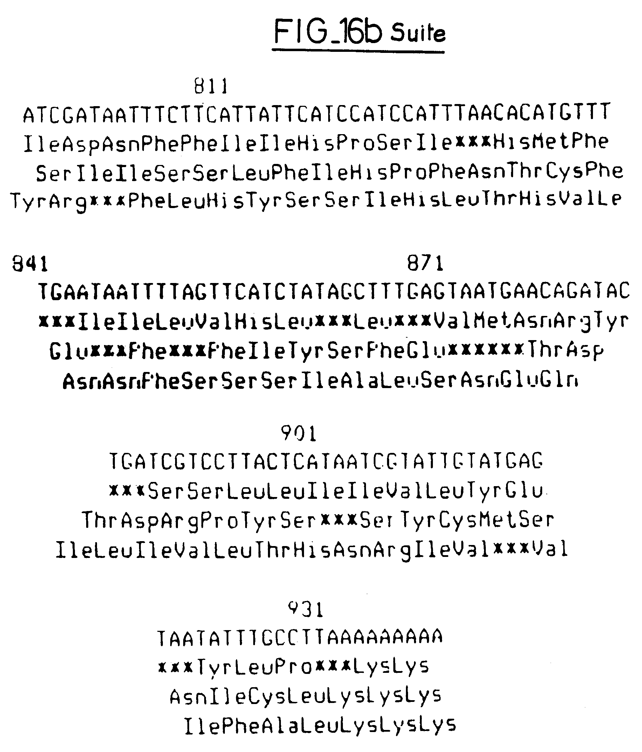

La séquence complète du cADN et la structure primaire en acides aminés qu'on peut en déduire (dans les 3 phases de lecture) sont présentées dans la figure 16a et 16b.

Exemple 9 Création d'un site BamHI au début de la séquence du cADN.

Pour faciliter l'insertion du cADN dans un vecteur d'expression approprié, on a créé, par mutagénèse dirigée, un site BamHI au début de la séquence codante.

La mutagénèse a été effectuée sur l'ADN cloné dans M13 avec l'oligomère de synthèse suivant, qui est un 21-mère complémentaire des nucléotides 18 à 38 de la séquence représentée dans la figure la, à l'exception des nucléotides à muter :

$$\downarrow$$
$$5' \text{ GTAAGGAGC}\underline{\text{GGATCC}}\text{ACGATG } 3'$$
$$\text{site BamHI}$$

La séquence de cADN peut donc être récupérée sous forme de fragment BamHI, un deuxième site BamHI étant présent dans le polylinker du M13, en aval de la séquence codante.

Exemple10 Expression de la protéine P28II dans E. coli.

La séquence de cADN codant pour la P28II a été récupérée sous forme de fragment BamHI et introduite dans le vecteur d'expression pTG908, dans le site BamHI.

La structure du recombinant obtenu est identique à celle qui est schématisée dans la figure 2.

Ce vecteur contient une origine de réplication, le promoteur $P_L$ du phage lambda et la séquence de fixation des ribosomes de cII incluant l'extrémité N-terminale du gène cII, avec un site de restriction BamHI 39 pb en aval du site d'initiation de la traduction.

L'insert de cADN-P28II se retrouve donc en phase avec l'extrémité N-terminale du gène cII.

Un plasmide recombinant portant l'insert dans la bonne orientation a été sélectionné : pTG56 et la construction a été vérifiée par séquençage.

Une culture de E. coli TGE901 transformée par ce plasmide est mise en culture sur milieu LB jusqu'à une $DO_{600}$ de 0,2 ; ensuite la synthèse de la protéine cII-P28II est induite par une augmentation de la température à 42°C pendant les 8 heures suivantes.

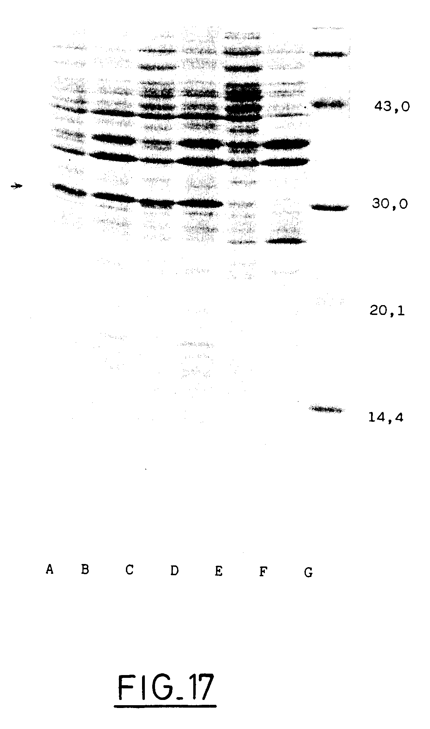

En fin de culture, les extraits cellulaires sont récupérés après traitement aux ultra-sons et analysés après électrophorèse sur gel d'acrylamide-SDS et coloration au bleu de Coomassie (Figure 17).

Une bande d'une masse moléculaire apparente de 28K est nettement visible dans les extraits de TGE901/pTG56 (Figure 17 : bandes A, B, C, D).

Une analyse par "Western blot" d'une préparation purifiée de cette protéine extraite de E. coli montre qu'elle est reconnue par les anticorps de rat dirigés contre la P28 native purifiée à partir des schistosomes.

Une éventuelle activité enzymatique de glutathion-S-transférase a été recherchée mais le test est négatif. Ce résultat négatif était attendu, étant donné l'absence d'homologie entre les séquences des 2 protéines P28.

Exemple 11 Vaccination de rats avec l'antigène P28II produit par E. coli.

Les rats sont immunisés selon le protocole décrit dans l'exemple 5 et leurs sérums sont évalués dans un test de cytotoxicité dépendante des éosinophiles.

Les résultats présentés dans le tableau IVmontrent que le pouvoir cytotoxique de ces sérums est comparable à celui des rats immunisés avec le premier antigène P28I ou avec la P28 native.

Tableau IV

| Test de cytotoxicité dépendante d'éosinophiles : étude des sérums de rats immunisés avec la protéine P28 native, cII-P28 et cII-P28II produites dans E. coli (dilution du sérum 1/16). | | | |
|---|---|---|---|
| rats immunisés avec : | % de cytotoxicité du sérum récolté au jour | | |
| | 7 | 14 | 21 |
| P28 native | 52,5 % | 29 % | 73 % |
| cII-P28I/coli | 24,5 % | 20 % | 59,5 % |
| cII-P28II/coli | 55 % | 68 % | 61,5 % |
| adjuvant seul (BSA + Pertusis) | | 30 % | |
| rats non injectés | | 0 % | |
| rats infectés par S. mansoni | | 89,5 % | |

Les anticorps des rats immunisés avec la protéine P28II produite dans E. coli ne reconnaissent pas la protéine P28I. De même, les anticorps des rats immunisés avec la protéine P28I produite dans E. coli ne reconnaissent pas la protéine P28II. Toutefois les 2 types d'anticorps reconnaissent la préparation de P28 native purifiée à partir des schistosomes et les anticorps dressés contre la P28 native reconnaissent les 2 protéines recombinantes.

Ce résultat s'explique par une hétérogénéité de la préparation de P28 native purifiée à partir de schistosomes. Une observation attentive de l'immunoprécipitation après migration sur gel en 2 dimensions de la protéine P28 native ou traduite in vitro sur les ARNm de schistosomes révèle l'existence d'une bande mineure qui dédouble la bande de 28 K (Figure publiée par J.M. Balloul, R.J. Pierce, J.M. Grzych et A. Capron, Mol. Biochem. Parasitology 17 (1985) 105-114). Cette bande mineure doit correspondre à la P28II.

Dépôt de souches

Les souches suivantes ont été déposées à la Collection Nationale de Culture de Microorganismes, 28 rue du Docteur Roux - 75724 PARIS :

La Souche de Ecoli N 4830 mentionnée dans l'exemple 2 transformée par le plasmide pTC44 a été déposée, sous le n° de dépôt I 562 le 6 Juin 1986.

La souche TGE901/pTG56 a été déposée à la Collection Nationale de Culture des Microorganismes, 25 rue du Docteur Rdux, PARIS, le 3 avril 1987, sous le n° I.656.

BIBLIOGRAPHIE

Capron, A. et Dessaint, J.P. (1985) Ann. Rev. Immunol. 3, 455-476

Balloul, J.M., Pierce, R.J., Grzych, J.M. et Capron, A. (1985) Mol. Biochem. Parasitol. 17, 105-114

Balloul, J.M., Grzych, J.M., Pierce, R.J. et Capron, A. (1986) Mol. Biochem. Parasitol. 18, 73-88.

Young, R.A. et Davis, R.W. (1983) Science 222, 778-782

Huynh, T.V. in DNA Cloning Techniques : A Practical Approach (Glover, D., Ed.) IRL Press, Oxford (1984)

Le Bouc, Y., Dreyer, D., Jaeger, F., Binoux, M. et Sondermeyer, P. (1986) FEBS Lett. 196, 108-112

Lathe, R. et Lecocq, J-P. (1977) Virology 83, 204-206

Hitzemann, R.A., Hagie, F.E., Hayflick, J.S., Chen, C.Y., Seeburg, P.H. et Derynck, R. (1982) Nucleic Ac. Res. 10, 7791-7808

Kieny, M.P., Lathe, R. et Lecocq, J-P. (1983) Gene 26, 91-99

Smith, G.L., Mackett, M. et Moss, B. (1983) Nature 302, 490-495

Panicalli, D. et Paoletti, E. (1982) Proc. Natl. Acad. Sci. USA 79, 4927-4931

Mackett, M., Smith, J.L. et Moss, B. (1982) Proc. Natl. Acad. Sci. USA 79, 7415-7419

Kieny, M.P., Lathe, R., Drillien, R., Spehner, D., Skory, S., Schmitt, D., Wiktor, T., Koprowski, H. et Lecocq, J-P. (1984) Nature 312, 163-166

Capron, M., Bazin, H., Torpier, G., Joseph, M. et Capron, A. (1981) J. Immunol. 126, 1764-1768

Habig, W.H., Pabst, M.J. & Jakoby, W.B. J. Biol. Chem. 249, 7130 (1974).

Moore, R.E., Davies, M.S., O'Connell, K.M., Harding, E.I., Wiegand, R.C. & Tiemeier, D.C. Nucl. Ac. Res. 14, 7227 (1986).

Smith, D.B., Davern, R.M., Board, P.G., Tiu, W.U., Garcia, E.G. & Mitchell, G.F. Proc. Natl. Acad. Sci. USA 83, 8703 (1986).

**Revendications**

**Revendications pour les Etats contractants suivants : AT, BE, CH, DE, FR, GB, IT, LI, LU, NL, SE**

1. Une protéine ayant une activité glutathion transférase et une séquence en acides aminés substantiellement telle que montrée à la Figure 1.

2. Une protéine ayant une activité glutathion transférase, et un poids moléculaire d'environ 28 Kd pouvant être obtenu à partir de Schistosoma mansoni.

3. Une protéine qui comprend les épitopes de la protéine selon la revendication 1 ou 2, qui sont reconnues par les anticorps dressés contre la protéine de la revendication 1 ou 2.

4. Un fragment d'ADN codant pour une protéine selon l'une des revendications 1 à 3.

5. Un bloc d'expression destiné à la production d'une protéine selon l'une des revendications 1 à 3, qui comprend un fragment d'ADN codant pour une protéine selon l'une des revendications 1 à 3, placé sous le contrôle d'éléments appropriés destinés à assurer l'expression dudit fragment d'ADN.

6. Une cellule transformée par un bloc d'expression selon la revendiction 5.

7. Un procédé de préparation d'une protéine selon l'une des revendications 1 à 3, qui comprend l'acte de cultiver une cellule selon la revendication 6 et de récolter ladite protéine à partir du milieu de culture.

8. Un vecteur viral destiné à la production d'une protéine selon l'une des revendications 1 à 3, dans le génome duquel est inséré un fragment d'ADN codant pour une protéine selon la revendication 1 ou 2, placé sous le contrôle des éléments d'expression appropriés.

9. Un vecteur viral selon la revendication 8 qui est un virus de la vaccine.

10. Une composition pharmaceutique destinée au traitement ou à la prévention de la schistosomiase qui comprend à titre d'agent thérapeutique une protéine selon l'une des revendications 1 à 3, ou un fragment immunogène de celle-ci comprenant les épitopes qui sont reconnues par les anticorps dressés contre la protéine.

11. Une composition pharmaceutique destinée au traitement ou à la prévention de la schistosomiase qui comprend à titre d'agent thérapeutique un vecteur viral selon la revendication 8 ou 9.

12. Application d'une protéine selon l'une des revendications 1 à 3, à titre d'agent présentant une activité glutathion transférase.

13. Application d'une cellule selon la revendication 6, à titre d'agent présentant une activité glutathion transférase.

14. Procédé de conversion microbiologique mettant en oeuvre une activité glutathion transférase caractérisée en ce que l'on utilise à titre d'agent de conversion une protéine selon l'une des revendications 1 à 3, ou une cellule selon la revendication 6.

**Revendications pour l'Etat contractant suivant: ES**

1. Procédé de préparation d'une protéine ayant une activité glutathion transférase caractérisée en ce qu'on synthétise ou purifie une séquence en acides aminés susbstantiellement telle que montrée à la Figure 1.

2. Procédé de préparation d'une protéine ayant une activité glutathion transférase, et un poids moléculaire d'environ 28 Kd obtenue à partir de <u>Schistosoma mansoni.</u>

3. Procédé de préparation d'une protéine qui comprend les épitopes de la protéine obtenue par le procédé selon la revendication 1 ou 2, à partir des anticorps dressés contre la protéine de la revendication 1 ou 2.

4. Procédé de préparation d'un fragment d'ADN caractérisé en ce qu'on synthétise une séquence codant pour une protéine selon l'une des revendications 1 à 3.

5. Procédé de préparation d'un bloc d'expression destiné à la production d'une protéine selon l'une des revendications 1 à 3, caractérisé en ce qu'un fragment d'ADN codant pour une protéine selon l'une des revendications 1 à 3, est placé sous le contrôle d'éléments appropriés destinés à assurer l'expression dudit fragment d'ADN.

6. Procédé de préparation d'une cellule transformée par un bloc d'expression selon la revendication 5.

7. Procédé de préparation d'une protéine selon l'une des revendications 1 à 3, qui comprend l'acte de cultiver une cellule selon la revendication 6 et de récolter ladite protéine à partir du milieu de culture.

8. Procédé de préparation d'un vecteur viral destiné à la production d'une protéine selon l'une des revendications 1 à 3, caractérisé en ce qu'on insère dans son génome un fragment d'ADN codant pour une protéine selon la revendication 1 ou 2, placé sous le contrôle des éléments d'expression appropriés.

9. Procédé de préparation d'un vecteur viral selon la revendication 8 qui est un virus de la vaccine.

10. Procédé de préparation d'une composition pharmaceutique destinée au traitement ou à la prévention de la schistosomiase caractérisé en ce qu'on utilise, à titre d'agent thérapeutique, une protéine selon l'une des revendications 1 à 3, ou un fragment immunogène de celle-ci comprenant les épitopes qui sont reconmus par les anticorps dressés contre la protéine.

11. Procédé de préparation d'une composition pharmaceutique destinée au traitement ou à la prévention de la shistosomiase caractérisé en ce qu'on utilise, à titre d'agent thérapeutique, un vecteur viral selon la revendication 8 ou 9.

12. Application d'une protéine selon l'une des revendications 1 à 3, à titre d'agent présentant une activité glutathion transférase.

13. Application d'une cellule selon la revendication 6, à titre d'agent présentant une activité glutathion transférase.

14. Procédé de conversion microbiologique mettant en oeuvre une activité glutathion transférase caractérisée en ce qu'on utilise, à titre d'agent de conversion, une protéine selon l'une des revendications 1 à 3, ou une cellule selonla revendication 6.

**Revendications pour l'Etat contractant suivant : GB**

1. Une protéine ayant une activité glutathion transférase et une séquence en acides aminés susbstantiellement telle que montrée à la Figure 1.

2. Une protéine ayant une activité glutathion transférase, et un poids moléculaire d'environ 28 Kd pouvant être obtenu à partir de Schistosoma mansoni.

3. Une protéine qui comprend les épitopes de la protéine selon la revendication 1 ou 2, qui sont reconnues par les anticorps dressés contre la protéine de la revendication 1 ou 2.

4. Un fragment d'ADN codant pour une protéine selon l'une des revendications 1 à 3.

5. Un bloc d'expression destiné à la production d'une protéine selon l'une des revendications 1 à 3, qui comprend un fragment d'ADN codant pour une protéine selon l'une des revendications 1 à 3, placé sous le contrôle d'éléments appropriés destinés à assurer l'expression dudit fragment d'ADN.

6. Une cellule transformée par un bloc d'expression selon la revendication 5.

7. Procédé de préparation d'une protéine selon l'une des revendications 1 à 3, qui comprend l'acte de cultiver une cellule selon la revendication 6 et de récolter ladite protéine à partir du milieu de culture.

8. Un vecteur viral destiné à la production d'une protéine selon l'une des revendications 1 à 3, dans le génome duquel est inséré un fragment d'ADN codant pour une protéine selon la revendication 1 ou 2, placé sous le contrôle des éléments d'expression appropriés.

9. Un vecteur viral selon la revendication 8 qui est un virus de la vaccine.

10. Procédé de préparation d'une composition pharmaceutique destinée au traitement ou à la prévention de la schistosomiase caractérisé en ce qu'on utilise, à titre d'agent thérapeutique, une protéine selon l'une des revendications 1 à 3, ou un fragment immunogène de celle-ci comprenant les épitopes qui sont reconmus par les anticorps dressés contre la protéine.

11. Procédé de préparation d'une composition pharmaceutique destinée au traitement ou à la prévention de la shistosomiase caractérisé en ce qu'on utilise, à titre d'agent thérapeutique, un vecteur viral selon la revendication 8 ou 9.

12. Application d'une protéine selon l'une des revendications 1 à 3, à titre d'agent présentant une activité glutathion transférase.

13. Application d'une cellule selon la revendication 6, à titre d'agent présentant une activité glutathion transférase.

14. Procédé de conversion microbiologique mettant en oeuvre une activité glutathion transférase caractérisée en ce qu'on utilise, à titre d'agent de conversion, une protéine selon l'une des revendications 1 à 3, ou une cellule selonla revendication 6.

**Claims**
**Claims for the following Contracting States : AT, BE, CH, DE, FR, GB, IT, LI, LU, NL, SE**

1. A protein having a glutathione transferase activity and an amino acid sequence substantially as shown in Figure 1.

2. A protein having a glutathione transferase activity and a molecular weight of about 28 Kd, which may be obtained from Schistosoma mansoni.

3. A protein which comprises the epitopes of the protein according to Claim 1 or 2, which are recognized by antibodies raised against the protein of Claim 1 or 2.

4. A DNA fragment encoding a protein according to one of Claims 1 to 3.

5. An expression unit intended for the production of a protein according to one of Claims 1 to 3, which comprises a DNA fragment encoding a protein according to one of Claims 1 to 3, placed under the control of appropriate elements intended to ensure the expression of the said DNA fragment.

6. A cell transformed by an expression unit according to Claim 5.

7. A method for preparing a protein according to one of Claims 1 to 3, which comprises the act of culturing a cell according to Claim 6 and harvesting the said protein from the culture medium.

8. A viral vector intended for the production of a protein according to one of Claims 1 to 3, in whose genome is inserted a DNA fragment encoding a protein according to Claim 1 or 2, placed under the control of appropriate expression elements.

9. A viral vector according to Claim 8, which is a vaccinia virus.

10. A pharmaceutical composition intended for the treatment or the prevention of schistosomiasis which comprises, as therapeutic agent, a protein according to one of Claims 1 to 3, or an immunogenic fragment thereof comprising the epitopes which are recognized by antibodies raised against the protein.

11. A pharmaceutical composition intended for the treatment or prevention of schistosomiasis which comprises, as therapeutic agent, a viral vector according to Claim 8 or 9.

12. Application of a protein according to one of Claims 1 to 3, as agent exhibiting a glutathione transferase activity.

13. Application of a cell according to Claim 6, as agent exhibiting a glutathione transferase activity.

14. Method of microbiological conversion using a glutathione transferase activity, characterized in that a protein according to one of Claims 1 to 3, or a cell according to Claim 6, is used as conversion agent.

**Claims for the following Contracting State : ES**

1. Method of preparing a protein having a glutathione transferase activity characterized in that an amino acid sequence substantially as shown in Figure 1 is synthesized or purified.

2. Method of preparing a protein having a glutathione transferase activity and a molecular weight of about 28 Kd, which is obtained from Schistosoma mansoni.

3. Method of preparing a protein which comprises the epitopes of the protein obtained by the method according to Claim 1 or 2, from antibodies raised against the protein of Claim 1 or 2.

4. Method of preparing a DNA fragment, characterized in that a sequence encoding a protein according to one of Claims 1 to 3, is synthesized.

5. Method of preparing an expression unit intended for the production of a protein according to one of Claims 1 to 3, characterized in that a DNA fragment encoding a protein according to one of Claims 1 to 3 is placed under the control of appropriate elements intended to ensure the expression of the said DNA fragment.

6. Method of preparing a cell transformed by an expression unit according to Claim 5.

7. Method of preparing a protein according to one of Claims 1 to 3, which comprises the act of culturing a cell according to Claim 6 and harvesting the said protein from the culture medium.

8. Method of preparing a viral vector intended for the production of a protein according to one of Claims 1 to 3, characterized in that a DNA fragment encoding a protein according to Claim 1 or 2, placed under the control of appropriate expression elements, is inserted into its genome.

9. Method of preparing a viral vector according to Claim 8, which is a vaccinia virus.

10. Method of preparing a pharmaceutical composition intended for the treatment or the prevention of schistosomiasis characterized in that a protein according to Claims 1 to 3, or an immunogenic fragment thereof comprising the epitopes which are recognized by antibodies raised against the protein, are used as therapeutic agent.

11. Method of preparing a pharmaceutical composition intended for the treatment or the prevention of schistosomiasis characterized in that a viral vector according to Claim 8 or 9 is used as therapeutic agent.

12. Application of a protein according to one of Claims 1 to 3, as agent exhibiting a glutathione transferase activity.

13. Application of a cell according to Claim 6, as agent exhibiting a glutathione transferase activity.

14. Method of microbiological conversion using a glutathione transferase activity, characterized in that a protein according to one of Claims 1 to 3, or a cell according to Claim 6, is used as conversion agent.

**Claims for the following Contracting State : GR**

1. A protein having a glutathione transferase activity and an amino acid sequence substantially as shown in Figure 1.

2. A protein having a glutathione transferase activity and a molecular weight of about 28 Kd, which may be obtained from Schistosoma mansoni.

3. A protein which comprises the epitopes of the protein according to Claim 1 or 2, which are recognized by antibodies raised against the protein of Claim 1 or 2.

4. A DNA fragment encoding a protein according to one of Claims 1 to 3.

5. An expression unit intended for the production of a protein according to one of Claims 1 to 3, which comprises a DNA fragment encoding a protein according to one of Claims 1 to 3, placed under the control of appropriate elements intended to ensure the expression of the said DNA fragment.

6. A cell transformed by an expression unit according to Claim 5

**7.** Method of preparing a protein according to one of Claims 1 to 3, which comprises the act of culturing a cell according to Claim 6 and harvesting the said protein from the culture medium.

**8.** A viral vector intended for the production of a protein according to one of Claims 1 to 3, in whose genome is inserted a DNA fragment encoding a protein according to Claim 1 or 2, placed under the control of appropriate expression elements.

**9.** A viral vector according to Claim 8, which is a vaccinia virus.

**10.** Method of preparing a pharmaceutical composition intended for the treatment or the prevention of schistosomiasis characterized in that a therapeutic agent, a protein according to one of Claims 1 to 3, or an immunogenic fragment thereof comprising the epitopes which are recognized by antibodies raised against the protein, are used as therapeutic agent.

**11.** Method of preparing a pharmaceutical composition intended for the treatment or the prevention of schistosomiasis characterized in that a viral vector according to Claim 8 or 9 is used as therapeutic agent.

**12.** Application of a protein according to one of Claims 1 to 3, as agent exhibiting a glutathione transferase activity.

**13.** Application of a cell according to Claim 6, as agent exhibiting a glutathione transferase activity.

**14.** Method of microbiological conversion using a glutathione transferase activity, characterized in that a protein according to one of Claims 1 to 3, or a cell according to Claim 6, is used as conversion agent.

**Patentansprüche**
**Patentansprüche für folgenden Vertragsstaat : AT, BE, CH, DE, FR, GB, IT, LI, LU, NL, SE**

**1.** Protein, das eine Glutathion-Transferase-Aktivität und eine Aminosäure-Sequenz, wie sie im wesentlichen in der Fig.1 dargestellt ist, aufweist.

**2.** Protein, das eine Glutathion-Transferase-Aktivität aufweist und ein Molekulargewicht von etwa 28 Kd hat, das aus Schistosoma mansoni gewonnen werden kann.

**3.** Protein, das die Epitope des Proteins nach Anspruch 1 oder 2 aufweist, die von gegen das Protein des Anspruchs 1 oder 2 gerichteten Antikörpern erkannt werden.

**4.** DNA-Fragment, das für ein Protein nach einem der Ansprüche 1 bis 3 codiert.

**5.** Expressions-Block für die Herstellung eines Proteins nach einem der Ansprüche 1 bis 3, der ein DNA-Fragment aufweist, das für ein Protein nach einem der Ansprüche 1 bis 3 codiert, das unter der Kontrolle von geeigneten Elementen steht, die dafür bestimmt sind, die Expression des genannten DNA-Fragments zu gewährleisten.

**6.** Zelle, die durch einen Expressions-Block nach Anspruch 5 transformiert worden ist.

**7.** Verfahren zur Herstellung eines Proteins nach einem der Ansprüche 1 bis 3, bei dem man eine Zelle nach Anspruch 6 züchtet und das genannte Protein aus dem Kulturmedium gewinnt.

**8.** Virus-Vektor für die Herstellung eines Proteins nach einem der Ansprüche 1 bis 3 in dem Genom, in das ein DNA-Fragment inseriert ist, das für ein Protein nach Anspruch 1 oder 2 codiert, der unter der Kontrolle von geeigneten Expressionselementen steht.

**9.** Virus-Vektor nach Anspruch 8, bei dem es sich um ein Vaccine-Virus handelt.

**10.** Pharmazeutische Zusammensetzung für die Behandlung oder die Prävention der Schistosomiasis, das als therapeutisches Agens ein Protein nach einem der Ansprüche 1 bis 3 oder ein immunogenes

23

Fragment desselben enthält, das die Epitope aufweist, die von den gegen das Protein gerichteten Antikörpern erkannt werden.

**11.** Pharmazeutische Zusammensetzung für die Behandlung oder die Prävention der Schistosomiasis, das als therapeutisches Agens einen Virus-Vektor nach Anspruch 8 oder 9 enthält.

**12.** Verwendung eines Proteins nach einem der Ansprüche 1 bis 3 als Agens, das eine Glutathion-Transferase-Aktivität aufweist.

**13.** Verwendung einer Zelle nach Anspruch 6 als Agens, das eine Glutathion-Transferase-Aktivität aufweist.

**14.** Mikrobiologisches Umwandlungsverfahren, bei dem eine Glutathion-Transferase-Aktivität angewendet wird, dadurch gekennzeichnet, daß man als Umwandlungsagens ein Protein nach einem der Ansprüche 1 bis 3 oder eine Zelle nach Anspruch 6 verwendet.

**Patentansprüche für folgenden Vertragsstaat : ES**

**1.** Verfahren zur Herstellung eines Proteins, das eine Glutathion-Transferase-Aktivität aufweist, dadurch gekennzeichnet, daß man eine Aminosäure-Sequenz, wie sie im wesentlichen in der Fig. 1 dargestellt ist, synthetisiert oder reinigt.

**2.** Verfahren zur Herstellung eines Proteins, das eine Glutathion-Transferase-Aktivität und ein Molekulargewicht von etwa 28 Kd aufweist, das aus Schistosoma mansoni gewonnen wurde.

**3.** Verfahren zur Herstellung eines Proteins, das die Epitope des durch das Verfahren nach Anspruch 1 oder 2 erhaltenen Proteins aufweist, aus Antikörpern, die gegen das Protein des Anspruchs 1 oder 2 gerichtet sind.

**4.** Verfahren zur Herstellung eines DNA-Fragments, dadurch gekennzeichnet, daß man eine Sequenz synthetisiert, die für ein Protein nach einem der Ansprüche 1 bis 3 codiert.

**5.** Verfahren zur Herstellung eines Expressions-Blockes für die Herstellung eines Proteins nach einem der Ansprüche 1 bis 3, dadurch gekennzeichnet, daß ein DNA-Fragment, das für ein Protein nach einem der Ansprüche 1 bis 3 codiert, unter die Kontrolle von geeigneten Elementen gestellt wird, die dazu bestimmt sind, die Expression des genannten DNA-Fragments zu gewährleisten.

**6.** Verfahren zur Herstellung einer Zelle, die durch einen Expressions-Block nach Anspruch 5 transformiert worden ist.

**7.** Verfahren zur Herstellung eines Proteins nach einem der Ansprüche 1 bis 3, bei dem man eine Zelle nach Anspruch 6 züchtet und das genannte Protein aus dem Kulturmedium gewinnt.

**8.** Verfahren zur Herstellung eines Virus-Vektors für die Herstellung eines Proteins nach einem der Ansprüche 1 bis 3, dadurch gekennzeichnet, daß man in sein Genom ein DNA-Fragment inseriert, das für ein Protein nach Anspruch 1 oder 2 codiert, das unter der Kontrolle geeigneter Expressionselemente steht.

**9.** Verfahren zur Herstellung eines Virus-Vektors nach Anspruch 8, bei dem es sich um ein Vaccine-Virus handelt.

**10.** Verfahren zur Herstellung einer pharmazeutischen Zusammensetzung für die Behandlung oder Prävention der Schistosomiasis, dadurch gekennzeichnet, daß man als therapeutisches Agens ein Protein nach einem der Ansprüche 1 bis 3 oder ein immunogenes Fragment desselben verwendet, das die Epitope aufweist, die von den gegen das Protein gerichteten Antikörpern erkannt werden.

**11.** Verfahren zur Herstellung einer pharmazeutischen Zusammensetzung für die Behandlung oder Prävention der Schistosomiasis, dadurch gekennzeichnet, daß man als therapeutisches Agens einen Virus-Vektor nach Anspruch 8 oder 9 verwendet.

**12.** Verwendung eines Proteins nach einem der Ansprüche 1 bis 3 als Agens, das eine Glutathion-Transferase-Aktivität aufweist.

**13.** Verwendung einer Zelle nach Anspruch 6 als Agens, das eine Glutathion-Transferase-Aktivität aufweist.

**14.** Mikrobiologisches Umwandlungsverfahren, bei dem eine Glutathion-Transferase-Aktivität angewendet wird, dadurch gekennzeichnet, daß man als Umwandlungsagens ein Protein nach einem der Ansprüche 1 bis 3 oder eine Zelle nach Anspruch 6 verwendet.

**Patentansprüche für folgenden Vertragsstaat : GR**

**1.** Protein, das eine Glutathion-Transferase-Aktivität und eine Aminosäure-Sequenz, wie sie im wesentlichen in der Fig. 1 dargestellt ist, aufweist.

**2.** Protein, das eine Glutathion-Transferase-Aktivität aufweist und ein Molekulargewicht von etwa 28 Kd hat, das aus Schistosoma mansoni gewonnen werden kann.

**3.** Protein, das die Epitope des Proteins nach Anspruch 1 oder 2 aufweist, die von gegen das Protein des Anspruchs 1 oder 2 gerichteten Antikörpern erkannt werden.

**4.** DNA-Fragment, das für ein Protein nach einem der Ansprüche 1 bis 3 codiert.

**5.** Expressions-Block für die Herstellung eines Proteins nach einem der Ansprüche 1 bis 3, der ein DNA-Fragment aufweist, das für ein Protein nach einem der Ansprüche 1 bis 3 codiert, das unter der Kontrolle von geeigneten Elementen steht, die dafür bestimmt sind, die Expression des genannten DNA-Fragments zu gewährleisten.

**6.** Zelle, die durch einen Expressions-Block nach Anspruch 5 transformiert worden ist.

**7.** Verfahren zur Herstellung eines Proteins nach einem der Ansprüche 1 bis 3, bei dem man eine Zelle nach Anspruch 6 züchtet und das genannte Protein aus dem Kulturmedium gewinnt.

**8.** Virus-Vektor für die Herstellung eines Proteins nach einem der Ansprüche 1 bis 3 in dem Genom, in das ein DNA-Fragment inseriert ist, das für ein Protein nach Anspruch 1 oder 2 codiert, der unter der Kontrolle von geeigneten Expressions elementen steht.

**9.** Virus-Vektor nach Anspruch 8, bei dem es sich um ein Vaccine-Virus handelt.

**10.** Verfahren zur Herstellung einer pharmazeutischen Zusammensetzung für die Behandlung oder Prävention der Schistosomiasis, dadurch gekennzeichnet, daß man als therapeutisches Agens ein Protein nach einem der Ansprüche 1 bis 3 oder ein immunogenes Fragment desselben verwendet, das die Epitope aufweist, die von den gegen das Protein gerichteten Antikörpern erkannt werden.

**11.** Verfahren zur Herstellung einer pharmazeutischen Zusammensetzung für die Behandlung oder Prävention der Schistosomiasis, dadurch gekennzeichnet, daß man als therapeutisches Agens einen Virus-Vektor nach Anspruch 8 oder 9 verwendet.

**12.** Verwendung eines Proteins nach einem der Ansprüche 1 bis 3 als Agens, das eine Glutathion-Transferase-Aktivität aufweist.

**13.** Verwendung einer Zelle nach Anspruch 6 als Agens, das eine Glutathion-Transferase-Aktivität aufweist.

**14.** Mikrobiologisches Umwandlungsverfahren, bei dem eine Glutathion-Transferase-Aktivität angewendet wird, dadurch gekennzeichnet, daß man als Umwandlungsagens ein Protein nach einem der Ansprüche 1 bis 3 oder eine Zelle nach Anspruch 6 verwendet.

TGCAAG

## Fig. 1

```
7
ATG GCT GGC GAG CAT ATC AAG GTT ATC TAT
Met Ala Gly Glu His Ile Lys Val Ile Tyr

37
TTT GAC GGA CGC GGA CGT GCT GAA TCG ATT
Phe Asp Gly Arg Gly Arg Ala Glu Ser Ile

67
CGG ATG ACT CTT GTG GCA GCT GGT GTA GAC
Arg Met Thr Leu Val Ala Ala Gly Val Asp

97
TAC GAA GAT GAG AGA ATT AGT TTC CAA GAT
Tyr Glu Asp Glu Arg Ile Ser Phe Gln Asp

127
TGG CCA AAA ATC AAA CCA ACT ATT CCA GGC
Trp Pro Lys Ile Lys Pro Thr Ile Pro Gly

157
GGA CGA TTG CCT GCA GTG AAA GTC ACT GAT
Gly Arg Leu Pro Ala Val Lys Val Thr Asp

187
GAT CAT GGG CAC GTG AAA TGG ATG TTA GAG
Asp His Gly His Val Lys Trp Met Leu Glu

217
AGT TTG GCT ATT GCA CGG TAT ATG GCG AAG
Ser Leu Ala Ile Ala Arg Tyr Met Ala Lys

247
AAA CAT CAT ATG ATG GGT GAA ACA GAC GAG
Lys His His Met Met Gly Glu Thr Asp Glu
```

# FIG.1

277
```
GAA  TAC  TAT  AGT  GTT  GAA  AAG  TTG  ATT  GGT
Glu  Tyr  Tyr  Ser  Val  Glu  Lys  Leu  Ile  Gly
```

307
```
CAG  GCT  GAA  GAT  GTA  GAA  CAT  GAA  TAT  CAC
Gln  Ala  Glu  Asp  Val  Glu  His  Glu  Tyr  His
```

337
```
AAA  ACT  TTG  ATG  AAG  CCA  CAA  GAA  GAG  AAA
Lys  Thr  Leu  Met  Lys  Pro  Gln  Glu  Glu  Lys
```

367
```
GAG  AAG  ATA  ACC  AAA  GAG  ATA  TTG  AAC  GGC
Glu  Lys  Ile  Thr  Lys  Glu  Ile  Leu  Asn  Gly
```

397
```
AAA  GTT  CCA  GTT  CTT  CTC  AAT  ATG  ATC  TGC
Lys  Val  Pro  Val  Leu  Leu  Asn  Met  Ile  Cys
```

427
```
GAA  TCT  CTG  AAA  GGG  TCG  ACA  GGA  AAG  CTG
Glu  Ser  Leu  Lys  Gly  Ser  Thr  Gly  Lys  Leu
```

457
```
GCT  GTT  GGG  GAC  AAA  GTA  ACT  CTA  GCT  GAT
Ala  Val  Gly  Asp  Lys  Val  Thr  Leu  Ala  Asp
```

487
```
TTA  GTC  CTG  ATT  GCT  GTC  ATT  GAT  CAT  GTG
Leu  Val  Leu  Ile  Ala  Val  Ile  Asp  His  Val
```

517
```
ACT  GAT  CTG  GAT  AAA  GGA  TTT  CTA  ACT  GGC
Thr  Asp  Leu  Asp  Lys  Gly  Phe  Leu  Thr  Gly
```

# FIG.1

547
```
AAG TAT CCT GAG ATC CAT AAA CAT CGA GAA
Lys Tyr Pro Glu Ile His Lys His Arg Glu
```

577
```
AAT CTG TTA GCC AGT TCA CCG CGT TTG GCG
Asn Leu Leu Ala Ser Ser Pro Arg Leu Ala
```

607
```
AAA TAT TTA TCG AAC AGG CCT GCA ACT CCC
Lys Tyr Leu Ser Asn Arg Pro Ala Thr Pro
```

637
```
TTC TAA ACC TAT CAA CAG AAA GCT CGG TGT
Phe ***
```

667
```
AAC GAG ATT TAA GAT ATT GAT AGT AAA GGA
```

697
```
CTA GTG TCA CCT TTT TAC AAG GAT GTC ATT
```

727
```
TGTTTTATGGGTGTTTTTTTCGCAATTG
```

757                                         787
```
TTATTAAAATTAACTTAGTTTCCTGTTTAAAAA
```

FIG_2

EP 0 268 501 B1

# FIG.3

## Nom de la séquence : (ALP)SM-CIIP28:SEQ

```
1
ATG GTT CGT GCA AAC AAA CGC AAC GAG GCT
Met Val Arg Ala Asn Lys Arg Asn Glu Ala

31
CTA CGA ATC GGA TCC GAA TTC CCC CCC CCA
Leu Arg Ile Gly Ser Glu Phe Pro Pro Pro

61
GAT TGG CCA AAA ATC AAA CCA ACT ATT CCA
Asp Trp Pro Lys Ile Lys Pro Thr Ile Pro

91
GGC GGA CGA TTG CCT GCA GTG AAA GTC ACT
Gly Gly Arg Leu Pro Ala Val Lys Val Thr

121
GAT GAT CAT GGG CAC GTG AAA TGG ATG TTA
Asp Asp His Gly His Val Lys Trp Met Leu

151
GAG AGT TTG GCT ATT GCA CGG TAT ATG GCG
Glu Ser Leu Ala Ile Ala Arg Tyr Met Ala

181
AAG AAA CAT CAT ATG ATG GGT GAA ACA GAC
Lys Lys His His Met Met Gly Glu Thr Asp

211
GAG GAA TAC TAT AGT GTT GAA AAG TTG ATT
Glu Glu Tyr Tyr Ser Val Glu Lys Leu Ile

241
GGT CAG GCT GAA GAT GTA GAA CAT GAA TAT
Gly Gln Ala Glu Asp Val Glu His Glu Tyr
```

# FIG.3

271
CAC AAA ACT TTG ATG AAG CCA CAA GAA GAG
His Lys Thr Leu Met Lys Pro Gln Glu Glu

301
AAA GAG AAG ATA ACC AAA GAG ATA TTG AAC
Lys Glu Lys Ile Thr Lys Glu Ile Leu Asn

331
GGC AAA GTT CCA GTT CTT CTC AAT ATG ATC
Gly Lys Val Pro Val Leu Leu Asn Met Ile

361
TGC GAA TCT CTG AAA GGG TCG ACA GGA AAG
Cys Glu Ser Leu Lys Gly Ser Thr Gly Lys

391
CTG GCT GTT GGG GAC AAA GTA ACT CTA GCT
Leu Ala Val Gly Asp Lys Val Thr Leu Ala

421
GAT TTA GTC CTG ATT GCT GTC ATT GAT CAT
Asp Leu Val Leu Ile Ala Val Ile Asp His

451                                                481
GTG ACT GAT CTG GAT AAA GGA TTT CTA ACT GGC
Val Thr Asp Leu Asp Lys Gly Phe Leu Thr Gly

                                          511
AAG TAT CCT GAG ATC CAT AAA CAT CGA GAA AAT
Lys Tyr Pro Glu Ile His Lys His Arg Glu Asn

                                   541
CTG TTA GCC AGT TCA CCG CGT TTG    GCG AAA
Leu Leu Ala Ser Ser Pro Arg Leu    Ala Lys

                              571
TAT TTA TCG AAC AGG CCT GCA ACT CCC TTC TAA
Tyr Leu Ser Asn Arg Pro Ala Thr Pro Phe ***

FIG. 4

FIG. 5

EP 0 268 501 B1

A  B  C  D  E  F  G

FIG_6

FIG_7

Pst I                                                    Pst I

pTG 188        | signal IL-2 |        IL-2        |

Ser . Ser . Ser . Thr . Lys . Lys . Thr . Gln

-------- TCG AGC TCG ACA AAG AAA ACA CAG --------

            ↓      ↓↓↓
            *      ***

Ser . Ser . Ser . Thr . Lys . Glu . Phe . Gln

pTG 188-I  -------- TCG AGC TCG ACA AAG GAA TTC CAG --------

FIG. 8

EP 0 268 501 B1

# Fig. 9

Nom de la séquence : (ALP)PTG45:SEQ
------------

AGATCTGCAGCA


13
ATG TAC CGC ATG CAA CTC CTG TCT TGT ATC
Met Tyr Arg Met Gln Leu Leu Ser Cys Ile


43
GCC TTA AGT CTC GCA CTT GTC ACA AAC AGC
Ala Leu Ser Leu Ala Leu Val Thr Asn Ser


73
GCT CCT ACT TCA AGC TCG ACA AAG GAA TTC
Ala Pro Thr Ser Ser Ser Thr Lys Glu Phe


103
CCC CCC CCA GAT TGG CCA AAA ATC AAA CCA
Pro Pro Pro Asp Trp Pro Lys Ile Lys Pro


133
ACT ATT CCA GGC GGA CGA TTG CCT GCA GTG
Thr Ile Pro Gly Gly Arg Leu Pro Ala Val


163
AAA GTC ACT GAT GAT CAT GGG CAC GTG AAA
Lys Val Thr Asp Asp His Gly His Val Lys


193
TGG ATG TTA GAG AGT TTG GCT ATT GCA CGG
Trp Met Leu Glu Ser Leu Ala Ile Ala Arg

# FIG.9

223
TAT ATG GCG AAG AAA CAT CAT ATG ATG GGT
Tyr Met Ala Lys Lys His His Met Met Gly

253
GAA ACA GAC GAG GAA TAC TAT AGT GTT GAA
Glu Thr Asp Glu Glu Tyr Tyr Ser Val Glu

283
AAG TTG ATT GGT CAG GCT GAA GAT GTA GAA
Lys Leu Ile Gly Gln Ala Glu Asp Val Glu

313
CAT GAA TAT CAC AAA ACT TTG ATG AAG CCA
His Glu Tyr His Lys Thr Leu Met Lys Pro

343
CAA GAA GAG AAA GAG AAG ATA ACC AAA GAG
Gln Glu Glu Lys Glu Lys Ile Thr Lys Glu

373
ATA TTG AAC GGC AAA GTT CCA GTT CTT CTC
Ile Leu Asn Gly Lys Val Pro Val Leu Leu

403
AAT ATG ATC TGC GAA TCT CTG AAA GGG TCG
Asn Met Ile Cys Glu Ser Leu Lys Gly Ser

433
ACA GGA AAG CTG GCT GTT GGG GAC AAA GTA
Thr Gly Lys Leu Ala Val Gly Asp Lys Val

# FIG.9

463
  ACT CTA GCT GAT TTA GTC CTG ATT GCT GTC
  Thr Leu Ala Asp Leu Val Leu Ile Ala Val

493
  ATT GAT CAT GTG ACT GAT CTG GAT AAA GGA
  Ile Asp His Val Thr Asp Leu Asp Lys Gly

523
  TTT CTA ACT GGC AAG TAT CCT GAG ATC CAT
  Phe Leu Thr Gly Lys Tyr Pro Glu Ile His

553
  AAA CAT CGA GAA AAT CTG TTA GCC AGT TCA
  Lys His Arg Glu Asn Leu Leu Ala Ser Ser

583
  CCG CGT TTG GCG AAA TAT TTA TCG AAC AGG
  Pro Arg Leu Ala Lys Tyr Leu Ser Asn Arg

613
  CCT GCA ACT CCC TTC TAA
  Pro Ala Thr Pro Phe ***

# Fig.10

Nom de la séquence:(ALP)PTG46:SEQ
--------------

AGATCTGCAGCA

13
ATG TAC CGC ATG CAA CTC CTG TCT TGT ATC
Met Tyr Arg Met Gln Leu Leu Ser Cys Ile

43
GCC TTA AGT CTC GCA CTT GTC ACA AAC AGC
Ala Leu Ser Leu Ala Leu Val Thr Asn Ser

73
GCT CCT ACT TCA AGC TCG ACA AAG GAA TTC
Ala Pro Thr Ser Ser Ser Thr Lys Glu Phe

103
CCC CCC CCC CAG GAA TAC TAT AGT GTT GAA
Pro Pro Pro Gln Glu Tyr Tyr Ser Val Glu

133
AAG TTG ATT GGT CAG GCT GAA GAT GTA GAA
Lys Leu Ile Gly Gln Ala Glu Asp Val Glu

163
CAT GAA TAT CAC AAA ACT TTG ATG AAG CCA
His Glu Tyr His Lys Thr Leu Met Lys Pro

193
CAA GAA GAG AAA GAG AAG ATA ACC AAA GAG
Gln Glu Glu Lys Glu Lys Ile Thr Lys Glu

# FIG.10

223
```
ATA TTG AAC GGC AAA GTT CCA GTT CTT CTC
Ile Leu Asn Gly Lys Val Pro Val Leu Leu
```

253
```
AAT ATG ATC TGC GAA TCT CTG AAA GGG TCG
Asn Met Ile Cys Glu Ser Leu Lys Gly Ser
```

283
```
ACA GGA AAG CTG GCT GTT GGG GAC AAA GTA
Thr Gly Lys Leu Ala Val Gly Asp Lys Val
```

313
```
ACT CTA GCT GAT TTA GTC CTG ATT GCT GTC
Thr Leu Ala Asp Leu Val Leu Ile Ala Val
```

343
```
ATT GAT CAT GTG ACT GAT CTG GAT AAA GGA
Ile Asp His Val Thr Asp Leu Asp Lys Gly
```

373
```
TTT CTA ACT GGC AAG TAT CCT GAG ATC CAT
Phe Leu Thr Gly Lys Tyr Pro Glu Ile His
```

403
```
AAA CAT CGA GAA AAT CTG TTA GCC AGT TCA
Lys His Arg Glu Asn Leu Leu Ala Ser Ser
```

433
```
CCG CGG GGG GGG GGG GGA ATT CTG TGA
Pro Arg Gly Gly Gly Gly Ile Leu ***
```

FIG.11

FIG. 12

FIG.13

FIG.14

FIG_15

# FIG_16a

GAATTCCCCCCCCCCCCCATCGTGGCATGGCTCCTTACAGAA
GluPheProProProProIleValAlaTrpLeuLeuThrGlu
AsnSerProProProProSerTrpHisGlySerLeuGln
IleProProProProHisArgGlyMetAlaProTyrArg

TTGTGTTTATTCGCCATGCAGAGACTGTTTACAAIG
LeuCysLeuPheAlaMetGlnArgValPheThrMet
AsnCysValTyrSerProCysArgGluCysLeuGln***
IleValPheIleArgHisAlaGluSerValTyrAsnGlu

AAGAAAATCCATTTTGTGCTTGGCATCATGCAGATCTTTCAG
LysLysIleAspPheValValGlyMetMetGlnIlePheGln
ArgLysSerIleLeuTrpLeuAla***CysArgSerPheArg
GluAsnArgPheCysGlyTrpHisAspAlaAspLeuSerGl

GACAAGGTATCACTGAGGCTAAACAAGCTGGCCAACTTCTAC
AspLysValSerLeuArgLeuAsnLysLeuAlaAsnPheTyr
ThrArgTyrHis***Gly***ThrSerTrpProThrSerThr
GlnGlyIleThrGluAlaLysGlnAlaGlyGlnLeuLeu

GCCAAAATCACTTCACCTTTGATATTGCCTATACAAGCG
AlaLysIleThrSerProLeuIleLeuProIleGlnAla
ProLysSerLeuHisLeu***TyrCysLeuTyrLys
ArgGlnAsnHisPheThrPheAspIleAlaTyrThrSer

# FIG.16a Suite

211
TTCTAAAAAGAGCCATCAAGACTTTAAACTTTGTCCTTG
Phe***LysGluProSerArgLeu***ThrLeuSerLeu
ArgSerLysLysSerHisGlnAspPheLysLeuCysPro***
ValLeuLysArgAlaIleLysThrLeuAsnPheValLeuAsp


241                                    271
ATGAACTTGATCTTAACTGGATACCTGTGACAAAAACATGGC
MetAsnLeuIleLeuThrGlyTyrLeu***GlnLysHisGly
xxxThrxxxSerxxxLeuAspThrCysAspLysAsnMet
GluLeuAspLeuAsnTrpIleProValThrLysThrTrp

301
GTCTAAATGAAAGAATGTACGGTGCTCTTCAAGGTCTG
Val***MetLysGluCysThrValLeuPheLysVal***
AlaSerLys***LysAsnValArgCysSerSerArgSer
ArgLeuAsnGluArgMetTyrGlyAlaLeuGlnGlyLeu

331
AATAAGTCTGAAACTGCTGCCAAACATGGAGAGGAACAAG
IleSerLeuLysLeuLeuProAsnMetGluArgAsnLys
Glu***Val***AsnCysCysGlnThrTrpArgGlyThrSer
AsnLysSerGluThrAlaAlaLysHisGlyGluGluGlnVal


361                                    391
TTAAAATATGGAGACGTGCTTATGATATACCTCCCCCTCCTG
LeuLysTyrGlyAspValLeuMetIleTyrLeuProLeuLeu
xxxAsnMetGluThrCysLeu***xxTyrThrSerProSerCys
LysIleTrpArgArgAlaTyrAspIleProProProProVal

# FIG_16a Suite

421

TTGACATTTCAGATCCTCGCTTCCCCGGTAATGAACCAA
LeuThrPheGlnIleLeuAlaSerProValMetAsnGln
xxxHisPheArgSerSerLeuProArgxxxxxxThr
AspIleSerAspProArgPheProGlyAsnGluPro

451

AGTATGCCTTACTTGACTCTTCCTGCATACCACGTACTG
SerMetProTyrLeuThrLeuProAlaTyrHisValLeu
LysValCysLeuThrxxxLeuPheLeuHisThrThrTyrxxx
LysTyrAlaLeuLeuAspSerSerCysIleProArgThrGlu

481                                  511

AGTGTTTAAAGGACACTGTTCAACGTGTACTGCCATTTTGGTTT
SerValxxxArgThrLeuPheAsnValTyrCysHisPheGlyLeu
ValPheLysGlyHisCysSerThrCysThrAlaIleLeuVal
CysLeuLysAspThrValGlnArgValLeuProPheTrpPhe

541

GATACTATTTCTGCAAGTATAAAGAGACGCGAACAGGTTCT
IleLeuPheLeuGlnValxxxArgAspAlaAsnArgPhex
xxxTyrTyrPheCysLysTyrLysGluThrArgThrGlySer
AspThrIleSerAlaSerIleLysArgArgGluGlnValLeu

571

GATTGTCGCCCATGGAAACAGTTTAAGAGCGCTTA
xxLeuSerProMetGluThrValxxxGluArgLeu
AspCysArgProTrpLysGlnPheLysSerAlaTyr
IleValAlaHisGlyAsnSerLeuArgAlaLeuIle

# FIG.16b

601                                    631
TCAAGTACTTGGATAATACATCTGATTCAGATATTGTGGACTCAA
SerSerThrTrpIleIleHisLeuIleGlnIleLeuTrpThrGln
GlnValLeuGly***TyrIle***PheArgTyrCysGlyLeu.
LysTyrLeuAspAsnThrSerAspSerAspIleValAspSer


661
TATACCCACTGGTATTCCACTAGTCTATGAAC
TyrThrHisTrpTyrSerThrSerLeu***
AsnIleProThrGlyIleProLeuValTyrGlu
IleTyrProLeuValPheHis***SerMetAsn


691
TGGATGCGAACTTGAAGCCAACCAAACACTATTATCTTGCCGA
ThrGlyCysGluLeuGluAlaAsnGlnThrLeuLeuSerCysArg
LeuAspAlaAsnLeuLysProThrLysHisTyrTyrLeuAlaAsp
TrpMetArgThr***SerGlnProAsnThrIleIleLeuProMe


721                                    751
TGAAGCGACAGTAGCAGCAGCAATAGCACGTGTGGCGAACCAGGG
***SerAspSerSerSerSerAsnSerThrCysGlyGluProGly
GluAlaThrValAlaAlaAlaIleAlaArgValAlaAsnGln
LysArgGln***GlnGlnGln***HisValTrpArgThrArg


781
AAAAAAGAAATGAAGCGAATAATTATCATT
LysLysGluMetLysArgIleIleIleIle
GlyLysLysLys***SerGlu***LeuSerLeu
GluLysArgAsnGluAlaAsnAsnTyrHis

# FIG_16b Suite

811
ATCGATAATTTCTTCATTATTCATCCATCCATTTAACACATGTTT
IleAspAsnPhePheIleIleHisProSerIle***HisMetPhe
SerIleIleSerSerLeuPheIleHisProPheAsnThrCysPhe
TyrArg***PheLeuHisTyrSerSerIleHisLeuThrHisValLe

841                              871
TGAATAATTTTAGTTCATCTATAGCTTTGAGTAATGAACAGATAC
***IleIleLeuValHisLeu***Leu***ValMetAsnArgTyr
Glu***Phe***PheIleTyrSerPheGlu*******ThrAsp
AsnAsnPheSerSerSerIleAlaLeuSerAsnGluGln

901
TGATCGTCCTTACTCATAATCGTATTGTATGAG
***SerSerLeuLeuIleIleValLeuTyrGlu
ThrAspArgProTyrSer***SerTyrCysMetSer
IleLeuIleValLeuThrHisAsnArgIleVal***Val

931
TAATATTTGCCTTAAAAAAAAA
***TyrLeuPro***LysLys
AsnIleCysLeuLysLysLys
IlePheAlaLeuLysLysLys

48

43,0

30,0

20,1

14,4

A    B    C    D    E    F    G

# FIG.17